# EUROPÄISCHE PATENTSCHRIFT

(11) **EP 2 094 864 B1**
(45) Veröffentlichungstag und Bekanntmachung des Hinweises auf die Patenterteilung: **21.03.2012**
(21) Anmeldenummer: 07857228.6
(22) Anmeldetag: 19.11.2007
(51) Int. Cl.: C12Q 1/68

(54) **VERWENDUNG VON GENVERÄNDERUNGEN IM MENSCHLICHEN GEN CHK1, DAS FÜR DIE CHECKPOINTKINASE 1 KODIERT**
USE OF GENETIC MODIFICATIONS IN HUMAN GENE CHK1 WHICH CODES FOR CHECKPOINT KINASE 1
UTILISATION DE MODIFICATIONS GÉNÉTIQUES DANS LE GÈNE HUMAIN CHK1 CODANT POUR LA KINASE POINT DE CONTRÔLE 1

(30) Priorität: 17.11.2006 DE 102006054292
(43) Veröffentlichungstag der Anmeldung: 02.09.2009
(73) Patentinhaber: Universität Duisburg-Essen, 45145 Essen (DE)
(72) Erfinder: RIEMANN, Kathrin, 45145 Essen (DE); SIFFERT, Winfried, 45884 Gelsenkirchen (DE)
(74) Vertreter: Kilger, Christian
(86) Internationale Anmeldenummer: PCT/EP2007/062519
(87) Internationale Veröffentlichungsnummer: WO 2008/059069

(56) Entgegenhaltungen:
- WO-A-99/11795
- WO-A-2005/118845
- US-A1- 2003 087 847
- STAMBROOK P: "Haplotypes, SNPs and disease" MUTATION RESEARCH, AMSTERDAM, NL, Bd. 554, Nr. 1-2, 4. Oktober 2004 (2004-10-04), Seiten 407-415, XP004534995 ISSN: 0027-5107
- RIEMANN KATHRIN ET AL: "Promoter polymorphisms in the gene encoding checkpoint kinase 1 are associated with survival in cancer" FASEB JOURNAL, Bd. 21, Nr. 5, April 2007 (2007-04), Seite A418, XP002483041 & EXPERIMENTAL BIOLOGY 2007 ANNUAL MEETING; WASHINGTON, DC, USA; APRIL 28 -MAY 02, 2007 ISSN: 0892-6638
- K. RIEMANN, C. HASENBEIN, G. WIEKER, H. RÜBBEN, K. W. SCHMID, G. ILIAKIS, W. SIFFERT: "CHK1 promoter polymorphism predicts survival in cancer" ASCO ANNUAL MEETING 2008, [Online] 2008, XP002483042 Gefunden im Internet: URL:www.abstract.asco.org/abstView_55_3068 5.html> [gefunden am 2008-06-05]

## Beschreibung

### TECHNISCHES GEBIET DER ERFINDUNG

Die Erfindung betrifft ein in vitro-Verfahren zur Vorhersage von Krankheitsrisiken, Krankheitsverläufen, Arzneimittelrisiken und zum Auffinden von Drug Targets.

### TECHNOLOGISCHER HINTERGRUND DER ERFINDUNG

Krebszellen sind gekennzeichnet durch einen Verlust der Kontaktinhibition und unkontrolliertes Zellwachstum. Ausgelöst werden solche Veränderungen spontan oder durch Noxen, so genannte Kanzerogene, welche das Erbgut schädigen. Zu solchen Noxen gehören viele Chemikalien, Tabakrauch, aber auch UV-Licht. Daneben spielen genetische Faktoren bei der Krebsentstehung eine herausragende Rolle. Kennzeichnend für Krebszellen ist neben ihrem ungehemmten Wachstum auch die Neigung, Metastasen in anderen Organen abzusiedeln.

Es ist von medizinisch überaus hoher Relevanz, Prognosefaktoren für den Verlauf von Krebserkrankungen zu definieren, die Auskunft über das Ansprechens auf bestimmte Therpapieformen geben oder die generell prädiktiv für das Auftreten von Metastasen, die Tumorprogression und das Überleben sind. Bislang werden in der Medizin dem Fachmann allgemein bekannte Prognosefaktoren eingesetzt. Hierzu gehören beispielsweise die Größe des Tumors, seine Eindringtiefe in das umgebende Gewebe, organüberschreitendes Wachstum, das Eindringen in Blut- oder Lymphgefäße oder in Lymphknoten, sowie der Differenzierungsgrad der Tumorzellen. Daneben existieren einige relativ unspezifische serologische Marker.

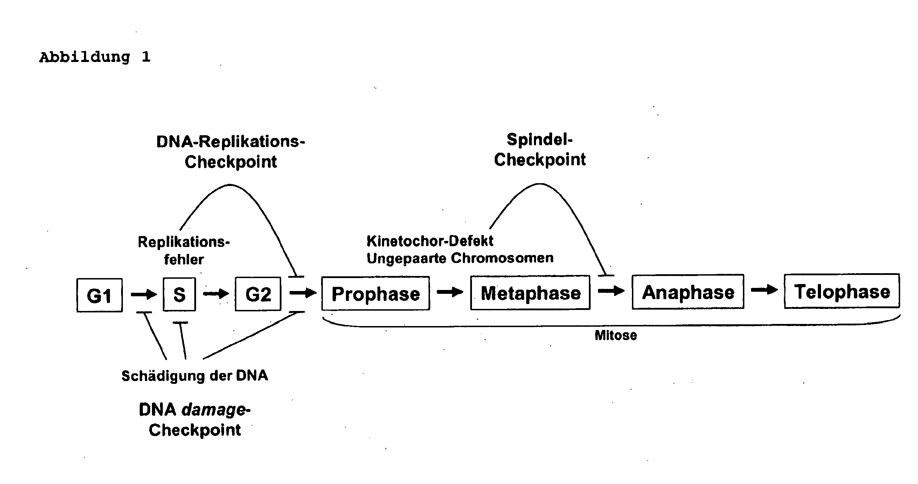

Der Zellzyklus der eukaryontischen Zellen ist allgemein in vier Phasen unterteilt: die G1-Phase, in der die Vorbereitung auf die Replikation stattfindet, die S-Phase, in der die DNA synthetisiert wird und die eigentlichen Zellfunktionen ablaufen, die G2-Phase, in der die Vorbereitung für die Mitose stattfindet, und in die M-Phase, die Mitose (Abbildung 1). Zusätzlich werden ausdifferenzierte Zellen, die sich nicht mehr teilen,' als sich in der G0-Phase befindend beschrieben. Dieses Organisationsprinzip ist zwar zweckmäßig, doch nach genauerer Betrachtung wird deutlich, dass der Zellzyklus weitaus komplexer ist. Zahlreiche Vorgänge müssen initiiert und aktiviert, einzelne Komponenten zusammengeführt und verschiedene Kaskaden koordiniert werden. Aus diesem Grund existieren diverse Kontrollmechanismen, die sicherstellen, dass sämtliche Prozesse innerhalb des Zellzyklus korrekt vollendet werden. Diese Kontrollmechanismen werden als "Checkpoints¹" bezeichnet. Dabei handelt es sich nicht um einen fest definierten Punkt, wie das Wort selbst impliziert, sondern um eine Reaktionskaskade, die unter bestimmten Umständen initiiert werden kann.
¹ Originaldefinition (nach Weinert et al. The RAD9 gene controls the cell cycle response to DNA damage in Saccharomyces cerevisiae. 1988 Science 241:317-22): Wenn ein Vorgang B abhängig von der Vervollständigung eines Vorganges A ist, dann wird diese Abhängigkeit von einem Checkpoint bedingt, sofern eine Mutation das Abhängigkeitsverhältnis aufheben kann.

Bisher wurden mehrere Zellzyklus-Checkpoints charakterisiert. Die am besten untersuchten Checkpoints in Säugetieren sind in Abbildung 1 dargestellt. Zum einen gibt es den DNA *damage*-Checkpoint, der durch Schädigung der DNA in unterschiedlichen Zellzyklus-Phasen aktiviert werden kann. Diese Schädigung kann sowohl durch exogene Ursachen, wie Strahlung, als auch durch endogene Vorgänge, z.B. spontane Mutationen, hervorgerufen werden. Zum anderen tritt durch eine nicht vollständige oder fehlerhafte Replikation der DNA der Replikations-Checkpoint in Kraft. Der Spindel-Checkpoint überwacht die Bildung der bipolaren Spindel, die Anlagerung der Kinetochore und die Neubildung der Zentromerstrukturen.

Solange diese Vorgänge nicht vollständig abgeschlossen sind oder die Schädigung nicht behoben ist, wird der Eintritt der Zelle in die nächste Zellzyklusphase inhibiert, um sicher zu stellen, dass die genomische Integrität der Zelle erhalten bleibt (Elledge SJ. Cell cylce checkpoints: preventing an identity crisis. 1996 Science 274:1664-72).

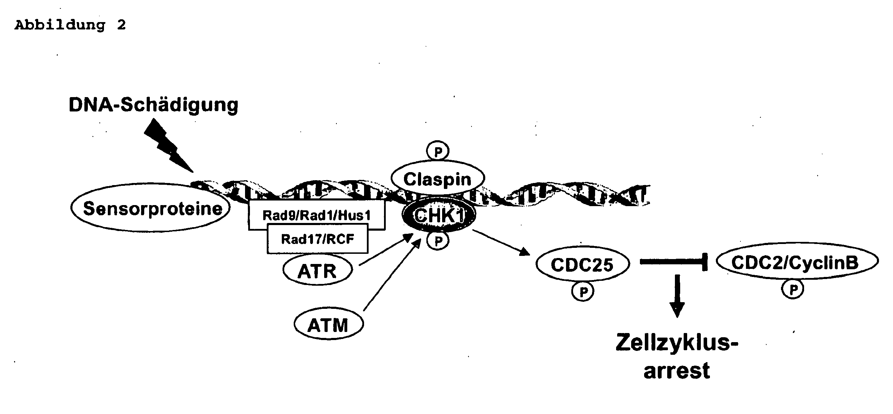

Die wichtigste Aufgabe einer Zelle ist, die genomische Integrität aufrecht zu erhalten. Die Checkpointkinase 1 ist in essentielle Kontrollmechanismen im Zellzyklus involviert, die sicherstellen, dass die Weitergabe von Fehlern an die Tochterzelle minimiert wird. Die maßgebliche CHK1-Reaktionskaskade am G2/M-Checkpoint ist in Abbildung 2 dargestellt. Die Aktivierung von CHK1 erfolgt aufgrund von DNA-Schädigungen, die hauptsächlich durch den Chromatin-gebundenen Rad17-Komplex erkannt werden. Der Radl7-Komplex rekrutiert daraufhin den Rad9-Hus1-Rad1-Komplex, der zusammen mit dem ATR-Atrip-Komplex CHK1, das teilweise Chromatin-assoziiert vorliegt, durch Phosphorylierung aktiviert. ATR (*Ataxia-telangiectasia and Rad3 related*) stellt hierbei die wichtigste aktivierende Komponente dar. Es wurde gezeigt, dass für die vollständige Aktivierung von CHK1 auch eine Phosphorylierung durch das Protein Claspin notwendig ist. Das aktivierte CHK1-Protein wandert aus dem Zellkern in das Zytoplasma, wo es CDC25C *(Cell division cycle 25C)* seinerseits durch Phosphorylierung aktiviert. Dieser Vorgang wiederum erlaubt dem 14-3-3-Protein (*Tyrosine 3-monooxygenase*/*tryptophan 5-monooxygenase activation protein*) an CHK1 zu binden, sodass sie in den Nukleus zurückkehren kann und dort verbleibt. Auf diese Weise wird CDC2 (*Cell division controller 2*) sowie der Cyclin B-Komplex inhibiert, was den Eintritt in die Mitose verhindert. Anschließend kann das DNA-Reparatur-System in Gang gesetzt werden, um die DNA-Schädigung zu beheben (Jiang et al. Regulation of Chk1 includes chrömatin association and 14-3-3 binding following phosphorylation on Ser345. 2003 J Biol Chem 278:25207-17; Jeong et al. Phosphorylated claspin interacts with a phosphate-binding site in the kinase domain of Chk1 during ATR-mediated activation. 2003 J Biol Chem 278:46782-8).

Für die CHK1 konnte auch eine Beteiligung an einem Checkpoint in der S-Phase nachgewiesen werden. Hier wird CHK1 bei einer fehlerhaften Replikation durch Phosphorylierung von ATM (*Ataxia-telangiectasia mutated*) aktiviert. Auch bei diesem Checkpoint ist eine zusätzliche Aktivierung durch Claspin erforderlich. Die vollständig aktivierte CHK1 aktiviert nun DNA-Proteinkinasen, mit denen sie zusammen p53 phosphorylieren und somit dessen Aktivität erhöhen kann. CHK1 ist ebenfalls in der Lage, TLK1 (*Tousled like kinase 1*) zu phosphorylieren. Dieses Protein spielt eine entscheidende Rolle bei der Chromatinkondensierung, die allerdings durch CHK1 gehemmt wird, um ein Fortschreiten im Zellzyklus zu verhindern. Weiterhin phosphoryliert CHK1 CDC25A (Cell division cycle 25A) und initiiert damit dessen Degradierung. Daraufhin ist das CDC-Protein nicht mehr in der Lage, die Cyclin-Komplexe zu aktivieren, wodurch weder die S-Phase vorangetrieben noch die M-Phase begonnen werden kann.

### ZUSAMMENFASSUNG DER ERFINDUNG

Der Erfindung liegt daher die Aufgabe zu Grunde, ein Mittel bereitzustellen, das eine bessere Prognose des natürlichen Verlaufs einer Krebserkrankung und das Ansprechen auf jedwede Therapieform erlaubt. Insbesondere soll dieses Mittel auch dazu in der Lage sein, diejenigen Patienten zu erkennen, bei denen gesteigerte DNA-Reparaturmechanismen eine Krebstherapie erschweren. Der Erfindung liegt ferner die Aufgabe zu Grunde, ein Mittel bereitzustellen, um generell Erkrankungsrisiken und Krankheitsverläufe vorherzusagen, da DNA-Reparaturmechanismen auch bei anderen Erkrankungen eine große Rolle spielen.

### Insbesondere sollen

(a)funktionsverändernde genomische Polymorphismen im Promotor des Gens *CHK1* bereitgestellt werden, die entweder zur Änderung der Proteinexpression oder zur Änderung der Expression von Spleißvarianten führen, oder
(b)die zum Auffinden und/oder Validieren weiterer Polymorphismen oder Haplotypen im Gen *CHK1* geeignet sind,
(c)Polymorphismen bereitgestellt werden, die geeignet sind, generell Krankheitsrisiken und -verläufe vorherzusagen,
(d)Polymorphismen bereitgestellt werden, die geeignet sind, generell Ansprechen auf Pharmaka und Krebstherapien, insbesondere CHK1-Inhibitoren, und Nebenwirkungen vorherzusagen,
(e)Polymorphismen bereitgestellt werden, die geeignet sind, generell die Wirkung anderer Therapieformen wie Bestrahlung, Wärme, Hitze, Kälte, Bewegung vorherzusagen.

Gelöst werden diese Aufgaben durch ein *in vitro*-Verfahren zur Vorhersage von Krankheitsrisiken und Krankheitsverläufen, einer Krebserkrankung , in dem man nach einer oder mehreren Genveränderung/en in der Promotorregion des Gens CHK1 (CHEK1) auf dem Humanchromosom 11q23 sucht, und die Genveränderung ausgewählt ist aus

(-1143)G>T, (-1400)C>T, (-1453) T>C und (-1454) insc; und durch ein In vitro-Verfahren zur Vorhersage des Ansprechens einer Radiochemotherapie bei einer Krebserkrankung dadurch gekennzeichnet, dass man nach der Genveränderung (-1143)G>T in der Promotorregion des Gens CHK1 (CHEK1) auf dem Humanchromosom 11q23 (Accession No. NM_033899 NCBI) sucht.

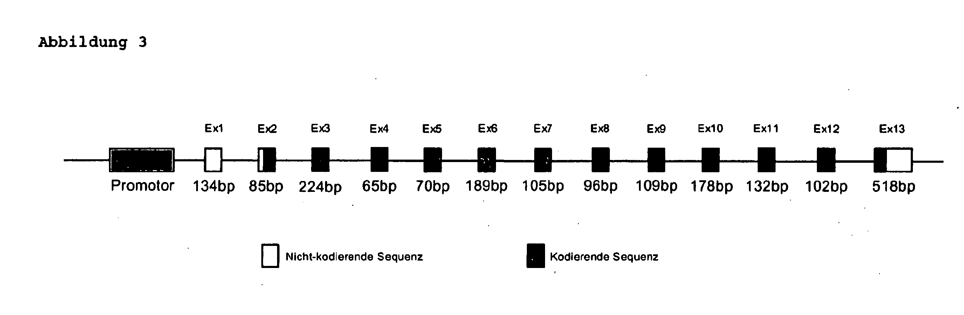

Das humane Gen *CHK1* ist auf Chromosom 11q23 lokalisiert (Accession no. NM_033899 der Genbank des National Center for Biotechnology Information (NCBI)) und kodiert für ein 54 kD großes Protein, das nukleär exprimiert wird. An dieser Stelle soll darauf hingewiesen werden, dass das Gen sowohl die Bezeichnung "*CHK1*" als auch die Bezeichnung "*CHEK1*" trägt. Im Folgenden wird hier die Bezeichnung "*CHK1*" verwendet. Eine schematische Darstellung der Genstruktur findet sich in Abbildung 3. Die aktive Promotorregion von *CHK1* wurde bereits charakterisiert. Die Promotorsequenz enthält zahlreiche Bindestellen für den Transkriptionsfaktor E2F1, dessen Bindung die transkriptionelle Aktivität verstärkt. Eine positive Regulation von *CHK1* wird ebenfalls durch eine Isoform des p53-abhängigen p73 beobachtet (Carrassa et al. Characterization of the 5' flanking region of the human Chk1 gene. 2003 Cell Cycle 2:604-9).

Abbildung 1 zeigt eine schematische Darstellung des Zellzyklus mit den wichtigsten Checkpoints.

Abbildung 2 zeigt eine graphische Darstellung der Reaktionskaskade am DNA *damage*-Checkpoint.

Abbildung 3 zeigt die Intron/Exon-Struktur des humanen Gens *CHK1* (nicht maßstabsgetreu).

Abbildung 4 zeigt die Strukturverwandtschaft einiger CHK1-Inhibitoren mit Staurosporin.

Abbildung 5 zeigt eine schematische Darstellung der Polymorphismen im Gen *CHK1* (nicht maßstabsgetreu).

Abbildung 6 zeigt die Kopplungsanalysen der Promotor-Polymorphismen von *CHK1* mit dem Programm Haploview; A-Graphische Darstellung der Kopplung der Polymorphismen untereinander. Schwarze Quadrate zeigen r²=1 an, graue Quadrate r²<0.5 und hellgraue Quadrate r²<0.1; B- Häufigkeiten und Kopplungsmöglichkeiten der einzelnen Allele; C- Häufigkeiten der konstruierten Haplotypen; Allele, die mit einem Dreieck gekennzeichnet sind, werden als sog. *haplotype-tagging alleles* bezeichnet, d. h. diese Allele müssen bestimmt werden, um die jeweiligen Haplotypen zu bestimmen.

Abbildung 7 zeigt putative Bindungsstellen für Transkriptionsfaktoren im Promotor des Gens *CHK1;* Die Zahlen auf der linken Seite repräsentieren den Bezug zum ATG.

Abbildung 8 zeigt-das Ergebnis des Electrophoretic Mobility Shift Assays (EMSA) mit Konstrukten, die die verschiedenen Allele des -1143G>T-Polymorphismus von *CHK1* enthalten. Nach Zugabe von Zellkernextrakt erkennt man eine vermehrte Bindung von Kernprotein and das "G-Konstrukt". Die Bindung wird durch die Gegenwart eines verdrängenden Oligonukleotids spezifisch gehemmt.

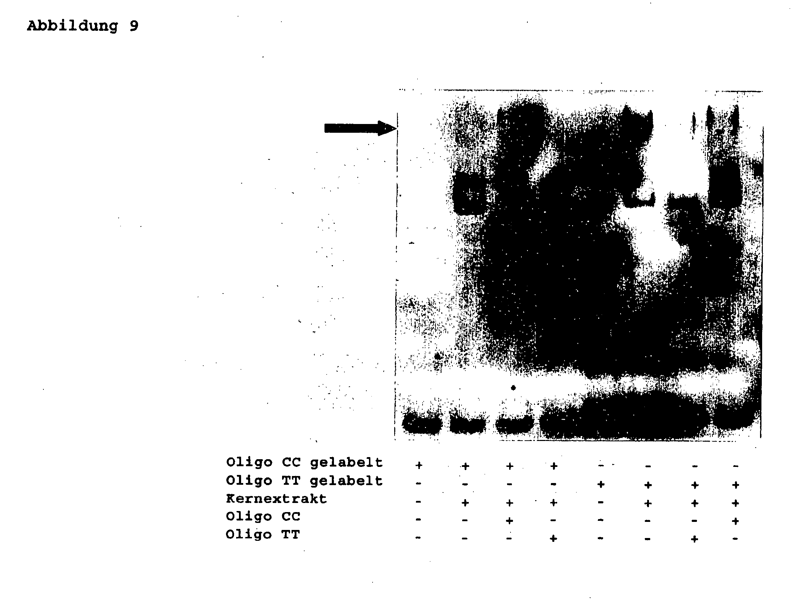

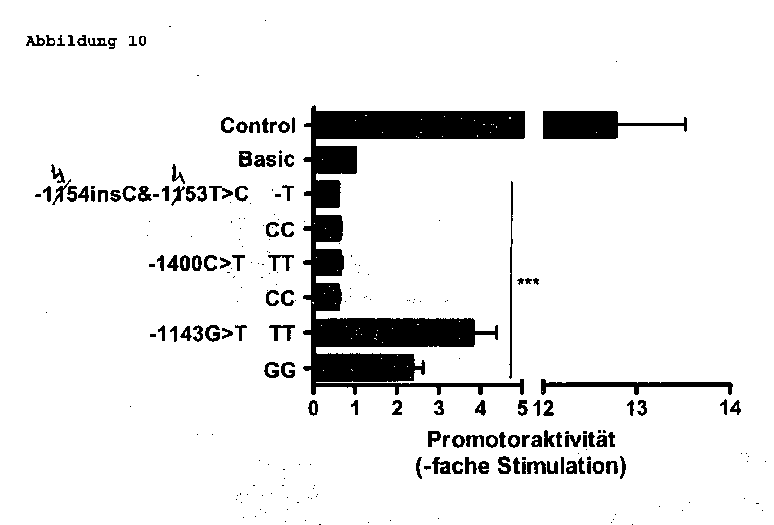

Abbildung 9 zeigt das Ergebnis des Electrophoretic Mobility Shift Assays (EMSA) mit Konstrukten, die die verschiedenen Allele des -1400C>T-Polymorphismus von *CHK1* enthalten. Nach Zugabe von Zellkernextrakt erkennt man eine vermehrte Bindung von Kernprotein and das "T-Konstrukt". Die Bindung wird durch die Gegenwart eines verdrängenden Oligonukleotids spezifisch gehemmt. Abbildung 10 zeigt Konstrukte zur Messung der Genotyp-abhängigen regulatorischen Aktivität der Promotor-Polymorphismen nach 24 h nach Transfektion von HELA-Zellen mittels sezernierter alkalischer Phosphatase (SEAP). Die Aktivität des -1143G>T-SNP ist signifikant höher als die Aktivität der anderen SNPs. ***: p<0.001.

Abbildung 11 zeigt eine Expression von cHK1-mRNA in Harnblasenkarzinom-Gewebe in Abhängigkeit der Allele der Promotor-Polymorphismen. Dargestellt ist der Quotient CHK1-/β-Actin-mRNA. A: -1143G>T, B: -1400C>T, C: -1453T>C und -1454insC; *_{:} p<0.05.

Abbildung 12 zeigt eine Expression von CHK1-mRNA in kolorektalem Karzinom-Gewebe in Abhängigkeit der Allele der Promotor-Polymorphismen. Dargestellt ist der Quotient CHK1-/β-Actin-mRNA. A: -1143G>T; B: -1400C>T, C: -1453T>C und -1454insC; *: p<0.05.

Abbildung 13 zeigt die Kaplan-Meier-Analyse zum Überleben von Patienten mit Harnblasenkarzinom abhängig vom Genotyp des -1143G>T-Polymorphismus. A: alle Patienten, B: nur die Patienten älter als 54 Jahre; *: p<0.05, **: p<0.01

Abbildung 14 zeigt die Kaplan-Meier-Analyse zum Überleben von Patienten mit kolorektalem Karzinom abhängig vom Genotyp der Promotor-Polymorphismen. A: Abhängigkeit vom - 1143G>T-SNP, B: nur Patienten mit Kolonkarzinom abhängig vom -1400C>T-SNP, C: nur die Patienten mit Kolonkarzinom abhängig von den -1453T>C- und -1454insC-SNPs; *: p<0.05, **: p<0.01

Abbildung 15 zeigt die Kaplan-Meier-Analyse zum Überleben von Patienten mit chronischer lymphatischer Leukämie abhängig vom Genotyp des -1143G>T-Polymorphismus.

Abbildung 16 zeigt die Kaplan-Meier-Analyse von Patienten mit Melanom abhängig vom Genotyp des -1400C>T-Promotor-Polymorphismus; A: Zeit von Ersttherapie bis zum Einsetzen der weiteren Therapie; B: Überleben; *: p<0.05.

Abbildung 17 zeigt die Kaplan-Meier-Analyse zum Überleben von Patienten mit cholangiozellulärem Karzinom abhängig vom Genotyp des -1400C>T-Polymorphismus; *: p<0.05.

Abbildung 18 zeigt eine Kaplan-Meier-Analyse zum Überleben von Patienten mit cholangiozellulärem Karzinom abhängig vom Genotyp des -1400C>T-Polymorphismus. *: p<0.05.

Abbildung 19 zeigt eine Kaplan-Meier-Analyse zum Überleben von Patientinnen mit Mammakarzinom abhängig vom Genotyp des -1143G>T-Polymorphismus. *: p<0.05

*CHK1* wird als potentielles Tumorsuppressor-Gen betrachtet, da ein Fehler in der Regulation des Zellzyklus zur Kumulation defekter DNA und einer erhöhten zellulären Proliferationsrate führt, was beides Charakteristika von Tumorzellen darstellt. Somatische Mutationen in diesem Gen konnten bisher in einigen Patienten mit sporadischen Tumoren nachgewiesen werden, z.B. wiesen Magen- und Mammakarzinome sowie Mikrosatelliten-instabile kolorektale Tumore Alterationen auf. Im Unterschied zu *single nucleotide polymorphisms* (SNPs) werden diese Mutationen bei den entsprechenden Patienten beispielsweise nicht in peripheren Blutzellen gefunden. Krankheits-spezifische Assoziationen zu SNPs wurden bisher noch nicht beschrieben.

Da Checkpoints in viele regulatorische Kaskaden involviert sind, sind sie ein geeignetes Ziel für Krebstherapeutika. Dazu tragen bestimmte Eigenschaften der Checkpoint-Proteine bei: (1) das komplexe Signaltransduktionssystem von Checkpoints bietet eine Vielzahl von Angriffszielen, (2) in gesunden Zellen scheinen einige Checkpoints relativ bedeutungslos zu sein, was die Toxizität der Inhibitoren stark reduziert, (3) die Restoration defekter Checkpoints könnte zu einer Verlangsamung des Zellwachstums führen, (4) als Signaltransduktionssystem unterliegen Checkpoints der Adaption, die unterbrochen werden könnte und (5) die Wiederherstellung beeinträchtigter Checkpoints könnte die Apoptoserate von Krebszellen erhöhen und somit ihre Sensitivität gegenüber bestimmten Stoffen erhöhen (Hartwell et al. Cell cycle control and cancer. 1994 Science 266:1821-8).

Im Gegensatz zu diesen Punkten, die am ehesten über einen Gentherapieansatz zu verwirklichen sind, stellen zwei weitere Eigenschaften von Checkpoints leichter realisierbare Angriffspunkte dar. Zellen mit defekten Checkpoints zeigen eine stark erhöhte Sensitivität gegenüber Strahlung und zytotoxischen Substanzen. Besonders der Verlust von CHK1 scheint Tumorzellen für diese Arten der Therapie zu prädisponieren.

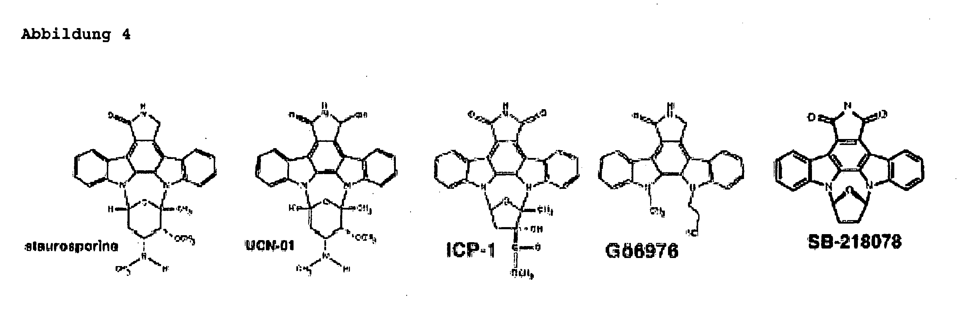

Diverse CHK1-Inhibitoren sind bereits bekannt oder entwickelt worden. Ausgehend vom ursprünglich als Proteinkinase C-Inhibitor identifizierten Staurosporin, das ebenfalls ein potenter CHK1-Inhibitor ist, wurden verschiedene Substanzen abgeleitet. Dazu gehören beispielsweise die CHK1-Inhibitoren UCN-01 (7-Hydroxystaurosporin), Gö6976, SB-218078, ICP-1, XL844 und CEP-3891, die den G2/M-Checkpoint blockieren (Abbildung 4). Ferner können Debromohymenialdisin und das synthetische Peptid TAT-S216A sowohl CHK1 als auch ein weiteres Checkpointprotein, CHK2, inhibieren. Es ist davon auszugehen, dass in naher Zukunft sogar noch weit mehr Substanzen zur Verfügung stehen werden, die die CHK1 hemmen

Unter den bisher zu Verfügung stehenden CHK1-Inhibitoren ist UCN-01 (7-Hydroxystaurosporin) die klinisch am besten charakterisierte Substanz. UCN-01 durchlief bereits mehrere klinische Studien der Phase I und wird derzeit in Phase II getestet. Die Hemmung oder Down-Regulation von CHK1 wirkte sich positiv auf das Ansprechen von Zytostatika, wie Topoisomerase-Inhibitoren, Antimitotika und Antimetaboliten aus, da durch das Fehlen von CHK1 die Toxizität dieser Chemotherapeutika besonders in aggressiven Tumoren, die auch nach vorangegangener herkömmlicher Therapie ein progredientes Wachstum gezeigt haben, potenziert wurde. Des Weiteren konnte gezeigt werden, dass UCN-01 die Sensitivität gegenüber Bestrahlung erhöht, wodurch CHK1-Inhibitoren auch das Potential als Radiosensitizer besitzen.

Wegen der grundlegenden Bedeutung von CHK1 für die Aufrechterhaltung der genomischen Integrität sind solche Polymorphismen geeignet, generell Erkrankungsrisiken oder Krankheitsverläufe bei Tumorerkrankungen vorherzusagen oder Therapieansprechen/Therapieversagen oder unerwünschte Nebenwirkungen für alle Pharmaka oder nichtpharmakologische Therapien vorherzusagen.

### Nachweis von Polymorphismen im Promotor des Gens CHK1

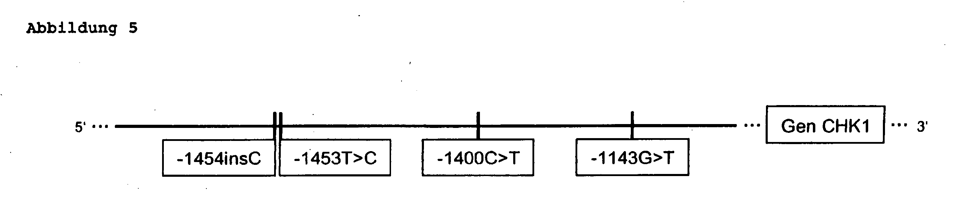

In der Promotorregion von *CHK1* sind fünf Polymorphismen bekannt und in allgemein zugänglichen Datenbanken aufzufinden. Durch die systematische Sequenzierung von DNA-Proben von Menschen wurden drei Polymorphismen nachgewiesen und validiert: -1143G>T (rs555752), -1400C>T (rs558351) und -1453T>C (rs1057733) (Abbildung 5). Hierzu wurden Gensequenzen des Promotorbereichs von *CHK1* mittels PCR-Reaktion amplifiziert und gemäß der Methode nach Sanger sequenziert. Die dazu erforderlichen Verfahren, z.B. das Ableiten von für die PCR-Reaktion erforderlichen Primerpaaren und die Auswahl von Sequenzier-Primern sind dem Fachmann geläufig. Hierbei wurde ein neuer Polymorphismus gefunden, wobei in der Promotorregion an Position -1454 eine Insertion von einem Cytosin vorliegt (-1154insC, keine dbSNP-Kennung vorhanden) (Abbildung 5). Die Nummerierung dieser SNPs erfolgt in der Weise, dass dem Nukleotid A des Startcodons ATG die Nummer +1 zugeordnet wird. Da es der Konvention entsprechend die Nummer 0 nicht gibt, ist dem vor dem A des Startcodons ATG liegenden Nukleotid die Nummer -1 zugeordnet.

Der Nachweis dieser, SNPs im Sinne ihrer erfindungsgemäßen Verwendung kann mit beliebigen, dem Fachmann geläufigen Verfahren unternommen werden, z.B. direkte Sequenzierung, PCR mit nachfolgender Restriktionsanalyse, reverse Hybridisierung, Dot-blot- oder Slot-blot-Verfahren, Massenspektrometrie, Taqman^{®}- oder Light-Cycler^{®}-Technologie, Pyrosequencing^{®}, Invader^{®}-Technologie, Luminex-Verfahren etc. Ferner können diese Genpolymorphismen gleichzeitig nach Mulitplex-PCR und Hybridisierung an einen DNA-Chip detektiert werden.

Es wurde die Verteilung der -1143G>T, -1400C>T, -1453T>C und -1454insC-Polymorphismen, Nachweis von Haplotypen und Verwendung dieser Genotypen zum Auffinden weiterer relevanter Polymorphismen und Haplotypen untersucht.

Hierzu wurden unterschiedliche DNA-Proben von Kaukasiern (n=205) genotypisiert. Das Ergebnis ist in der folgenden Tabelle dargestellt:

| **SNP** | **Genotypen** | | |
|---|---|---|---|
| -1143G>T | GG: 136 | GT: 65 | TT: 4 |
| -1400C>T | CC: 44 | CT: 107 | TT: 54 |
| -1453T>C | TT: 102 | TC: 85 | CC: 18 |
| -1454insC | - - : 102 | -C: 85 | CC: 18 |

Neben Kaukasiern wurde ebenfalls die Genotypverteilungen in Schwarzafrikanern überprüft:

| **SNP** | **Genotypen** | | |
|---|---|---|---|
| -1143G>T | GG: 57 | GT: 40 | TT: 5 |
| -1400C>T | CC: 61 | CT: 38 | TT: 2 |
| -1453T>C | TT: 54 | TC: 38 | CC: 4 |
| -1454insC | - - : 54 | -C: 38 | CC: 4 |

Die Genotypverteilungen in Chinesen sind in folgender Tabelle dargestellt:

| **SNP** | **Genotypen** | | |
|---|---|---|---|
| -1143G>T | GG: 72 | GT: 22 | TT: 4 |
| -1400C>T | CC: 37 | CT: 46 | TT: 16 |
| -1453T>C | TT: 30 | TC: 41 | CC: 15 |
| -1454insC | -- : 30 | -C: 41 | CC: 15 |

Ein Vergleich der Genotypverteilungen ergab in den meisten Fällen signifikante Unterschiede zwischen den Ethnien. Solche Unterschiede bei den Genotypverteilungen bei unterschiedlichen Ethnien weisen in der Regel darauf hin, dass assoziierte Phänotypen für die Evolution bedeutsam waren und den Trägern einen bestimmten Vorteil brachten. Es ist dem Fachmann bekannt, dass ethnisch unterschiedliche Genotypverteilungen ein Hinweis darauf sind, dass auch heute noch bestimmte Genotypen und Haplotypen mit bestimmten Erkrankungen oder physiologischen und pathopyhsiologischen Reaktionsweisen oder Ansprechen auf Therapie, z.B. mit Pharmaka, assoziiert sind.

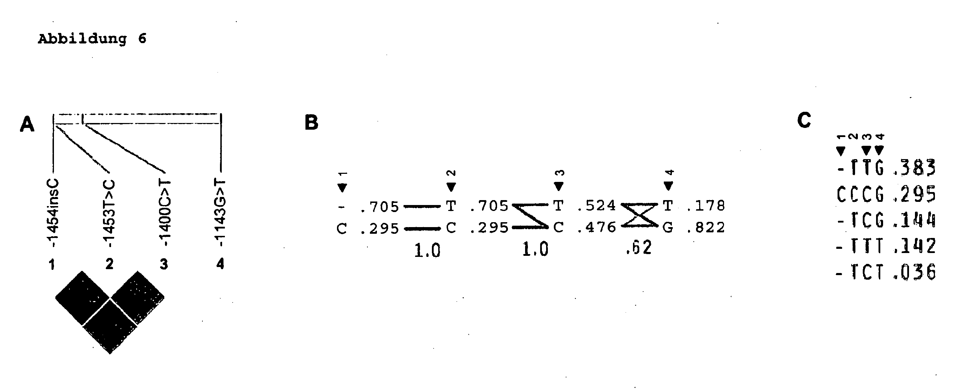

Weitere Analysen zeigten bei einhundert sequenzierten DNA-Proben von gesunden Kaukasiern ein Kopplungsungleichgewicht zwischen bestimmten Polymorphismen. Unter Kopplungsungleichgewicht versteht man das Auftreten von Allelkombinationen (Haplotypen), die statistisch eindeutig häufiger oder seltener gemeinsam vorkommen, als dies bezogen auf ihre Frequenz zu erwarten wäre. Dabei stellte sich heraus, dass die Polymorphismen -1453T>C und -1454insC vollständig aneinander koppeln. Die Polymorphismen -1143G>T und -1400C>T dagegen koppeln nicht aneinander und nur eingeschränkt an die beiden anderen Varianten (Abbildung 6A und B). Die Güte der Kopplung wird durch die Werte D' und r² gekennzeichnet. Dabei spricht man bei D'=1 und r²=1 von einer signifikanten Kopplung. Je näher beide Werte bei 1 liegen, desto enger ist das Kopplungsungleichgewicht. Die Berechnung der Haplotypen, die aus diesen vier Polymorphismen konstruiert werden können, ergab fünf verschiedene Allelkombinationen. Es existiert kein präferentieller Haplotyp, der sich aus diesen Promotorvarianten ergibt (Abbildung 6C). Um alle möglichen Kombinationen zu bestimmen, ist der Nachweis von mindestens drei der vier Polymorphismen notwendig.

Ein Gegenstand der Erfindung ist es, dass diese neuen Polymorphismen dazu benutzt werden können um weitere relevante genomische Genveränderungen in *CHK1* oder benachbarten Genen zu detektieren und zu validieren, die beispielsweise mit Genotypen im Gen *CHK1* im Kopplungsungleichgewicht stehen. Dies können auch Gene sein, die ebenfalls auf Chromosom 11 liegen, aber in großer Entfernung vom Gen *CHK1.* Hierzu wird folgendermaßen vorgegangen:
1. Für bestimmte Phänotypen (zelluläre Eigenschaften, Krankheitszustände, Krankheitsverläufe, Arzneimittelansprechen usw.) wird zunächst eine Assoziation mit den Polymorphismen -1453T>C, -1454insC, -1143G>T und -1400C>T hergestellt, wobei diese Assoziationen für jeden Genotyp einzeln oder unter Verwendung aller Permutationen der Haplotypen aufgestellt werden können.
2. Für neu detektierte Genveränderungen in *CHK1* oder benachbarten Genen wird untersucht, ob bereits bestehende Assoziationen unter Verwendung oben beschriebener Geno- oder Haplotypen verstärkt oder abgeschwächt werden.

### Funktionelle Bedeutung der Promotor-Polymorphismen im Gen CHK1

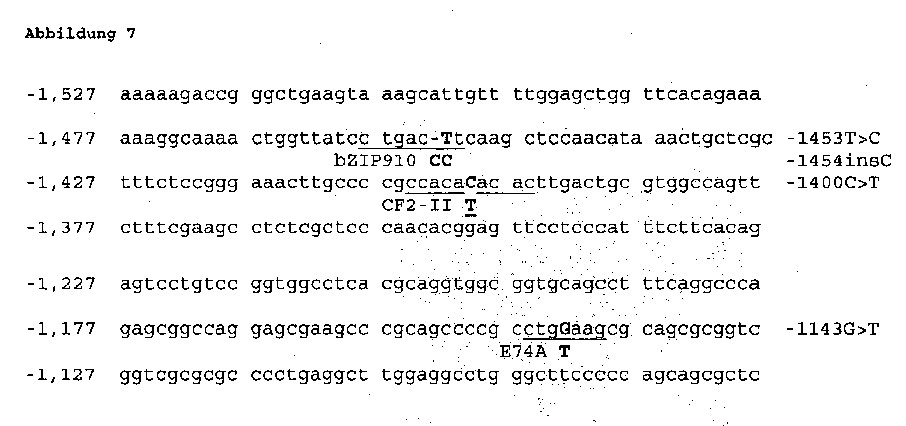

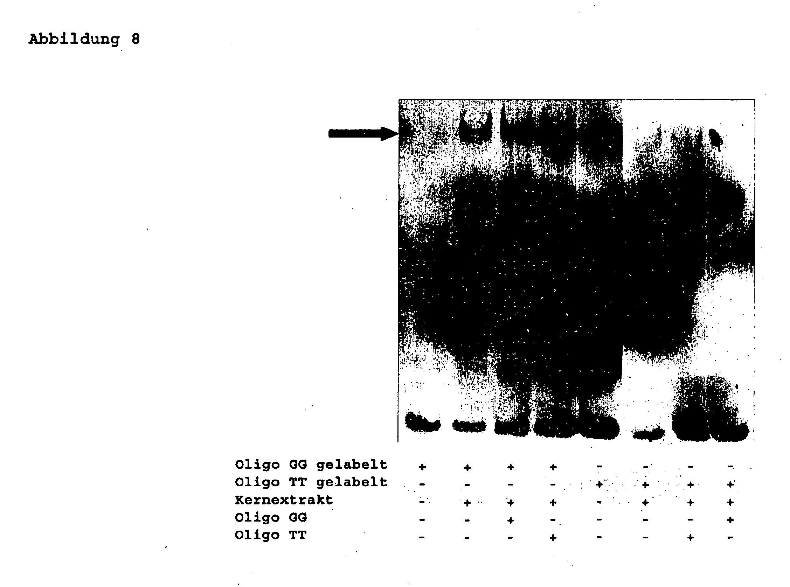

Es wurde untersucht, welche funktionellen Änderungen den Promotorpolymorphismen im Gen *CHK1* zuzuordnen sind. Denkbar sind hier beispielsweise eine Korrelation zu alternativem Spleißen, gewebespezifische Expression oder eine Überexpression des CHK1-Proteins in Abhängigkeit von Genotypen bzw. Haplotypen des Promotors von *CHK1.* Hierzu wurde zunächst mittels eines Computer-Programms untersucht, ob die gefundenen Nukleotidaustausche die Bindung von Transkriptionsfaktoren beeinflussen können. Transkriptionsfaktoren binden an spezifische Konsensus-Sequenzen und können die Promoteraktivität steigern oder vermindern, so dass eine verstärkte oder verminderte Transkription des Gens resultiert und somit das Expressionsniveau des kodierten Proteins gesteigert oder vermindert wird. Wie in Abbildung 7 dargestellt, befinden sich alle oben erwähnten Promotor-SNPs in einer Konsensus-Sequenz für Bindungsstellen unterschiedlicher Transkriptionsfaktoren (z.B. E74A, CF2-II oder bZIP910), deren Bindung durch die Polymorphismen beeinträchtigt werden kann. Das Auftreten bestimmter Genotypen führt zu einem Wegfall dieser Bindungsstellen durch die Veränderung ihrer Konsensus-Sequenzen. Zur experimentellen Untersuchung dieses Effekts wird ein so genannter EMSA (electrophoretic mobility shift assay) durchgeführt. Bei diesem Versuch werden kurze Nukleinsäureabschnitte, die den jeweiligen Polymorphismus beinhalten, mit Zellkernextrakten inkubiert. Transkriptionsfaktor-Proteine, die sich in diesen Extrakten befinden, binden nun mit unterschiedlicher Intensität an die Nukleinsäureabschnitte. Die Bindung an die DNA wird schließlich im Röntgenfilm sichtbar gemacht. Dabei resultiert aus einer starken Bindung eine intensive Bande. Abbildung 8 zeigt das Resultat dieses Versuches mit spezifischen Konstrukten, die entweder das G- oder das T-Allel des -1143G>T-Polymorphismus enthalten. Das Vorhandensein der G-Konstrukt-Bande beweist eine Bindung eines Transkriptionsfaktors an diese Region. Das T-Konstrukt weist keine Bande auf, was zeigt, dass kein Transkriptionsfaktor an dieses Allel bindet. Die Abschwächung der Bandenintensität durch ein spezifisches Oligonukleotid zeigt, dass es sich bei dem bindenden Transkriptionsfaktor um eine spezifische Bindung handelt. Abbildung 9 zeigt das Resultat dieses Versuches mit spezifischen Konstrukten, die entweder das C- oder das T-Allel des -1400C>T-Polymorphismus enthalten. Nur das T-Konstrukt führt zur Bindung eines Transkriptionsfaktors, während das C-Konstrukt keine spezifische Bande aufweist, demnach an diesem Allel auch kein Transkriptionsfaktor bindet. Die Verdrängung der Bande durch ein spezifisches Oligonukleotid zeigt, dass es sich bei dem bindenden Transkriptionsfaktor um eine spezifische Bindung handelt.

Zum funktionellen Nachweis einer regulatorischen Aktivität dieser Promotor-Regionen wurden abhängig von bestimmten Genotypen unterschiedliche Fragmente des Promotors in den Vektor pSEAP kloniert, um nach Expression des Vektors in HeLa-Zellen, einer Cervixkarzinom-Zellline, die regulatorische Aktivität mittels eines so genannten Reporterassays zu quantifizieren (Abbildung 10). Hierzu werden die Konstrukte vor ein Gen kloniert, das für sezernierte alkalische Phosphatase (*SEAP*) kodiert. Falls das Konstrukt eine Gen-regulierende Aktivität besitzt, wird das *SEAP*-Gen vermehrt transkribiert und die vermehrte Sekretion von alkalischer Phosphatase ins Zellkulturmedium ist messbar. Wie Abbildung 10 zeigt, weisen die Konstrukte mit den Allelen des -1143G>T-Polymorphismus eine signifikant höhere Aktivität auf als die Konstrukte der anderen Polymorphismen (p=0.0005). Die Reporter-Aktivität der einzelnen Allele dieses SNPs ist ebenfalls verschieden. Das T-Allel weist eine höhere Aktivität (3.82±0.6) auf als das G-Allel (2.36±0.3).

Da nur der -1143G>T-Polymorphismus des *CHK1*-Gens eine Reporter-Aktivität aufwies, wurde als nächstes überprüft, wie die Regulation *in vivo* stattfindet, weil es sich bei Reporter-Assays um ein artifizielles Zellsystem handelt. Dazu wurde die Expression von *CHK1* auf mRNA-Ebene mittels Realtime-PCR in menschlichem Gewebe untersucht.

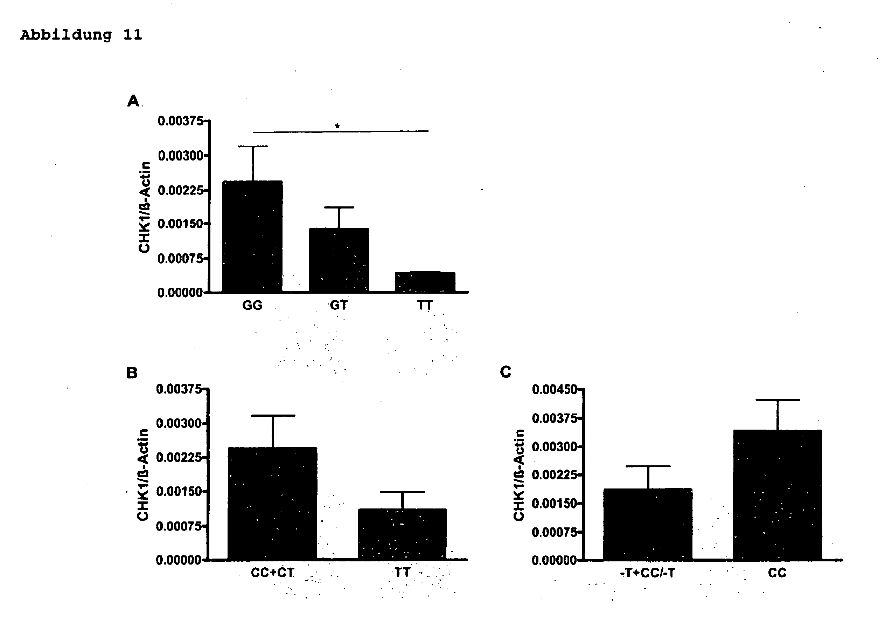

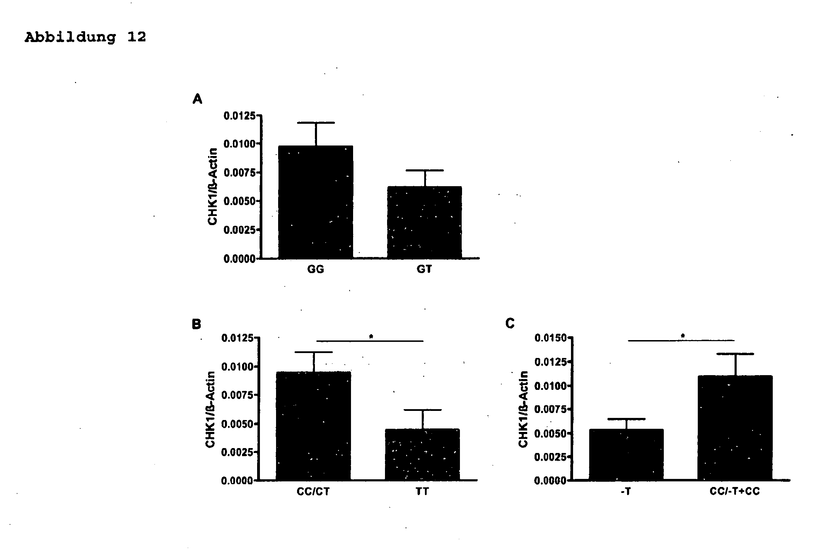

Hierfür wurde mRNA aus menschlichem Operationsgewebe bei Harnblasen- und Kolonoperationen gewonnen und mittels reverser Transkriptase in cDNA umgeschrieben. Das Verfahren ist dem Fachmann geläufig. Nachfolgend wurde das Expressionsniveau mittels Realtime-PCR (Taqman-Verfahren) bestimmt und mit dem Expressionsniveau des Housekeeping-Gens *β-Actin* abgeglichen. Die Ergebnisse sind in den Abbildungen 11 und 12 dargestellt. Teilabbildung 11A zeigt, dass der GG-Genotyp des -1143G>T-SNPs eine signifikant höhere mRNA-Expression aufweist als der TT-Genotyp. Die Werte des heterozygoten Genotyps liegen dazwischen, was für einen Gen-Dosis-Effekt spricht. Auch Abbildung 12A zeigt eine vermehrte mRNA-Expression für den GG-Genotyp. Auch die beiden anderen Polymorphismen zeigen einen Allel-abhängigen Unterschied in der Genexpression. Wie Abbildungen 11B und 12B veranschaulichen, haben C-Allel-Träger des -1400C>T-Polymorphismus deutlich mehr CHK1-mRNA als Träger des TT-Genotyps. Die Real time-PCR-Ergebnisse für die SNPs -1453T>C und -1454insC sind in den Abbildungen 11C und 12C dargestellt. Träger des T-Allels, die keine zusätzliche Insertion besitzen, zeigen eine deutlich niedrigere mRNA-Expression.

Damit wurde nachgewiesen, dass es im Gen *CHK1* Genveränderungen gibt, die eine Expressionsänderung von CHK1 im Karzinom-Gewebe bewirken. Dies können die oben beschriebenen Promotor-Polymorphismen sein oder Polymorphismen, die mit diesen SNPs im Kopplungsungleichgewicht stehen. Bestandteil.der hier beschrieben Erfindung ist damit auch, die Expression von CHK1 zu quantifizieren, mit bekannten Polymorphismen des *CHK1* zu assoziieren und neue, noch besser geeignete Polymorphismen zu entdecken und zu validieren.

Die hier gezeigten Befunde einer Genotyp-abhängigen Expression von CHK1 in humanem Karzinomgewebe ist überaus bedeutsam, da eine geringere Aktivität von CHK1 Mikrosatelliten- und chromosomale Instabilität hervorrufen kann, die beide mit zu den Kennzeichen genomischer Instabilität gehören und damit die Onkogenese begünstigen und sich negativ auf die Tumorprogression auswirken (Durkin et al. Depletion of CHK1, but not CHK2, induces chromosomal instability and breaks at common fragile sites. 2007 Oncogene 25:4381-8; Furlan et al. Genetic progression in sporadic endometrial and gastrointestinal cancers with high microsatellite instability. 2002 J Pathol 197:603-9). Zudem kann sich diese Genotyp-abhängige Genexpression von *CHK1* auch auf das Ansprechen auf eine Therapie mit CHK1-Inhibitoren auswirken. Es ist zu erwarten, dass eine niedrige Genexpression prädisponiert durch einen bestimmten Genotyp, z.B. der TT-Genotyp des -1143G>T-Polymorphismus, stärker auf CHK1-Inhibitoren anspricht als andere Genotypen. Damit können Genveränderungen im Gen CHK1 dazu verwendet werden, das Ansprechen auf eine Krebstherapie vorherzusaaen, um zum Beispiel Responder versus Non-Responder zu diskriminieren. Diese Genveränderungen können auch für eine Dosisfindung eingesetzt werden bzw. für die Vorhersage des Auftretens unerwünschter Arzneimittelwirkungen. Solche Krebstherapien können im weitesten Sinne medikamentös erfolgen, d.h. durch Zuführung von Substanzen in den Körper oder diese Krebstherapeutika können physikalisch wirksam sein (Bestrahlung, Wärme, Kälte).

Wir erwarten somit die Beeinflussung von Krankheitsverläufen, insbesondere bei Tumorerkrankungen, sowie ein geändertes Ansprechen auf Substanzen, die die Regulationskaskade von CHK1 beeinflussen oder Substanzen, die direkt CHK1 inhibieren.

### Verwendung von Genveränderungen in CHK1 zur Vorhersage von Erkrankungsrisiken und Krankheitsverläufen

Aufgrund der Schlüsselfunktion der Checkpointkinase 1 für die Regulation des Zellzyklus ist es ein wesentlicher Bestandteil der Erfindung, dass unter Verwendung von Genveränderungen in *CHK1* generell Erkrankungsrisiken und Krankheitsverläufe vorhergesagt werden können.

Die Multistep-Entwicklung von Krebs spiegelt die Akkumulation genetischer Veränderungen wider, die zur Transformation von normalen Zellen zu Krebszellen und von normalem Gewebe zu benignen und evtl. malignen, invasiven Tumoren führen. Die Ansammlung von Alterationen in Tumorsuppressorgenen und Protoonkogenen beschleunigt die Tumorgenese und kann sowohl Radio- als auch Chemotherapie beeinflussen. Es wird allerdings immer deutlicher, dass gestörte DNA-Reparaturmechanismen sowie Checkpoints der Grund für die erhöhte genomische Instabilität von Tumoren sind (Hoeijmaker JH. Genome maintenance mechanisms for preventing cancer. 2001 Nature 411:366-74; Khanna et al. DNA double-strand breaks: signaling, repair and the cancer connection. 2001 Nat Gent 27:247-54). Da Checkpoints eine zentrale Rolle für die Erhaltung der genomischen Integrität besitzen, ist unmittelbar zu erwarten, dass der Verlauf vielfältiger und ganz unterschiedlicher Tumorerkrankungen bei einer genetisch determinierten, verminderten Aktivierbarkeit von Checkpoints beeinflusst wird. Dies bedeutet, dass durch Expressionsänderungen von Proteinen, die in allen menschlichen Körperzellen exprimiert werden und die Zelle vor DNA-Schäden bewahren, Zellfunktionen reguliert werden, die alle physiologischen und pathophysiologischen Vorgänge entscheidend beeinflussen oder zumindest modulieren. Daneben werden auch Antworten auf Pharmaka in besonderer Weise beeinflusst. Hiervon sind erwünschte, aber auch unerwünschte Arzneimittelwirkungen betroffen.

Es wurde in der wissenschaftlichen Literatur wiederholt postuliert, dass Funktionsveränderungen von CheckpointProteinen einen nachhaltigen Einfluss auf vielfältige Erkrankungen bzw. auf den Verlauf vielfältiger Erkrankungen haben, da es sich um phylogenetisch hochkonservierte Pathways handelt. Solche Genveränderungen können strukturverändernde Mutationen in den Checkpointproteinen sein, die beispielsweise die Aktivierung der Proteine durch Phosphorylierung oder die Substratselektivität verringern. Ferner kann das Expressionsniveau verändert sein, wodurch die Initiation der nachfolgenden Reaktionskaskaden, die z. B. Apoptose induzieren, reduziert ist oder es können Spleißvarianten mit geänderter Funktion auftreten. All diese Veränderungen werden als genetische Prädisposition für Krebs angesehen.

Aus den genannten Beispielen geht folgendes hervor:
1. Genveränderungen in Genen, die für ubiquitär exprimierte Proteine kodieren, beeinflussen vielfältige Erkrankungen bzw. verursachen vielfältige Erkrankungsrisiken
2. Checkpoint-Proteine sind Teil des komplexen Netzwerkes zur Aufrechterhaltung der genomischen Integrität im menschlichen Körper

Erkrankungen, die mit einer Genveränderung in *CHK1 ein*hergehen und beispielsweise durch ein geändertes Expressionsniveau des CHK1-Proteins bestimmt werden, sind benigne Neoplasien jedes Ursprungsgewebes und maligne Neoplasien jedes Ursprungsgewebes.

Solche Neoplasien umfassen beispielsweise Tumorerkrankungen wie
Tumoren des Urogenitaltrakts: Hier sind zu nennen das Harnblasenkarzinom, das Nierenzellkarzinom, das Prostatakarzinom und das Seminom;
Tumoren der weiblichen Geschlechtsorgane: Das Mammakarzinom, das Korpuskarzinom, das Ovarialkarzinom, das Zervixkarzinom;
Tumoren des Gastrointestinaltraktes: Das Mundhöhlenkarzinom, das Ösophaguskarzinom, das Magenkarzinom, das Leberkarzinom, das Gallengangskarzinom, das Pankreaskarzinom, das Kolonkarzinom, das Rektumkarzinom;
Tumore des Respirationstraktes: das Kehlkopfkarzinom, das Bronchialkarzinom;
Tumore der Haut: das maligne Melanom, das Basaliom, das T-Zell-Lymphom;
Tumorerkrankungen des blutbildenden Systems: Hodgkin- und Non-Hodgkin-Lymphome, akute und chronische Leukämien, Plasmozytom;
Tumorerkrankungen des Gehirns bzw. des Nervengewebes: Glioblastom, Neuroblastom, Medulloblastom, meningeales Sarkom, Astrozytom;
Weichteiltumore, beispielsweise Sarkome und Kopf-Hals-Tumore.

### Verwendung von Genveränderungen im Gen CHK1 zur Vorhersage von Krankheitsverläufen und Therapieansprechen

Da die essentiellen Funktionen von CHK1 bekannt sind, können Genveränderungen im Gen *CHK1* das Risiko für Tumorerkrankungen erhöhen oder Krankheitsverläufe beeinflussen. Es ist generell nicht möglich, alle Tumorerkrankungen des Menschen und deren Verläufe zu untersuchen. Wir haben dies hier jedoch exemplarisch für fünf unterschiedliche Karzinome gezeigt: Harnblasenkarzinom, kolorektales Karzinom, chronische lymphatische Leukämie, malignes Melanom und cholangiozelluläres Karzinom. Diese Daten belegen eindeutig die Verwendbarkeit der Genveränderungen im Gen *CHK1* für den hier beschriebenen Zweck. Diese Erkrankungen stehen *a priori* in keinerlei Zusammenhang.

### Die Bedeutung der Checkpointkinase 1 für Chemotherapeutika und Bestrahlung

Genetische Instabilität ist ein Kennzeichen von allen Tumoren und spielt auch bei der Onkogenese, Progression und der Entwicklung von Resistenzen gegenüber Pharmaka eine Rolle (Hartwell L. Defects in a cell cycle checkpoint may be responsible for the genomic instability of cancer cells. 1992 Cell 71:543-6). Die meisten Tumorzellen besitzen einen defekten G1-S-Checkpoint, was ihnen einen Überlebensvorteil bringt. Dieser Defekt allerdings bedingt, dass Tumorzellen sehr abhängig vom G2-Checkpoint sind, wenn Stimuli vorhanden sind, die die genomische Integrität bedrohen. Die Hemmung des G2-Checkpoints durch Gabe von DNA-schädigenden Substanzen kann zu einer sog. "mitotischen Katastrophe", also Zelltod, führen. CHK1 ist verantwortlich für die Aufrechterhaltung des G2-Checkpoints, wenn DNA-Schäden auftreten. Somit bietet die Hemmung von CHK1 durch den Wegfall des G2-Checkpoints die Möglichkeit, dass sich-DNA-Schäden und -Veränderungen ansammeln können, die durch genotoxische Substanzen und Bestrahlung bedingt werden, und die Tumorzelle daran stirbt. Das setzt allerdings voraus, dass die Hemmung von CHK1 den somatischen Zelltod nicht fördert, was allgemeine zelluläre Toxizität und wenig Tumorspezifität bedeuten würde. Der Einsatz von CHK1-siRNA *in vitro* hat gezeigt, dass die CHK1-Hemmung nur wenige Auswirkungen auf den Zellzyklus und das Überleben der Zellen besitzt solange keine DNA-schädigenden Substanzen vorhanden sind. Beim Einsatz dieser Substanzen allerdings wird der G2-Checkpoint sicher gehemmt und die Apoptose gesteigert Seit der Entdeckung und Entwicklung der CHK1-Inhibitoren konnte bewiesen werden, dass durch deren Einsatz die Wirkung chemo- und radiotherapeutischer Maßnahmen gesteigert werden konnte

Treten Genveränderungen in *CHK1* auf, die die Genexpression beeinflussen, so hat dies Auswirkungen auf die Wirksamkeit dieser CHK1-Inhibitoren. Es ist zu erwarten, dass Patienten mit einer Genotyp-abhängigen niedrigeren CHK1-Expression besser auf die Inhibitoren ansprechen als Patienten mit einer höheren *CHK1*-Expression. Zudem bedeutet es, dass die Kombinationstherapie von CHK1-Inhibitoren mit Chemo- und Immuntherapeutika und/oder Bestrahlung beeinflusst werden kann. Daraus ergibt sich die Möglichkeit einer individuellen Diagnostik der generellen Ansprechbarkeit auf diese Krebstherapeutika und Therapiemaßnahmen sowie eine individuelle Vorhersage des Risikos unerwünschter Wirkungen durch diese Therapien.

Die Genotyp-abhängige Diagnostik der Expression von *CHK1* erlaubt eine generelle Diagnostik der Wirksamkeit von Chemotherapeutika und Bestrahlung, deren optimale Dosierung und das Auftreten von Nebenwirkungen.

Die Chemotherapie verwendet solche Stoffe, die ihre schädigende Wirkung möglichst gezielt auf bestimmte Krebszellen ausüben und diese abtöten oder in ihrem Wachstum hemmen. Eine bestimmte Zytostatikadosis kann immer nur einen bestimmten Anteil der Zielzellen töten, der mit fortschreitender Behandlung gleich bleibt. Deshalb darf eine Chemotherapie im Laufe, der Behandlung nicht vermindert werden, auch wenn der Tumor bereits nicht mehr nachweisbar ist. Es muss vielmehr damit gerechnet werden, dass durch eine schwache Behandlung gerade die widerstandsfähigen Tumorzellklone selektiert werden. Die Chemotherapie wird in schneller Abfolge appliziert, und fast immer werden zwei oder mehr Zytostatika kombiniert, um die Wirksamkeit zu erhöhen. Die Therapie ruft dadurch auch Nebenwirkungen hervor, die nach den Common Toxicity Criteria klassifiziert werden. Diese Kriterien beinhalten: Anzahl der Leukozyten und Thrombozyten, Übelkeit, Erbrechen, Durchfall und Stomatitis.

Unter Radiotherapie versteht man die Anwendung ionisierender hochenergetischer Strahlen, um bösartige Tumorerkrankungen zu heilen. Solche maligne Tumoren werden oft auch kombiniert chemo- und radiotherapeutisch behandelt. Eine Vielzahl an Tumorerkrankungen kann so auch in fortgeschrittenen Stadien geheilt werden. Um die Nebenwirkungen gering zu halten, wird die Bestrahlung auf viele tägliche Einzeldosen aufgeteilt und über mehrere Wochen verabreicht. Trotzdem treten Nebenwirkungen, wie Rötungen Übelkeit, Durchfälle oder Haarausfall, in Abhängigkeit von Dosis, Eindringtiefe und Anzahl der Einzeldosen auf. Der Erfindung liegt nun zugrunde, dass ein Verfahren entwickelt wurde, das generell zur Diagnostik der Aktivierbar der Checkpointkinase 1 und damit verbunden dem G2-Checkpoint geeignet ist. Hierzu werden eine oder mehrere Polymorphismen im Gen *CHK1* untersucht. Bei hohen Expression kommt es vorhersagbar zu einer gesteigerten Aktivierbarkeit des G2-Checkpoints und damit zu ausreichend Zeit, um Reparaturmechanismen an der DNA nach Schädigung derselben durchzuführen. Bei einer niedrigen *CHK1-*Expression ist der G2-Checkpoint weniger aktivierbar und DNA-Schäden werden nicht oder nicht ausreichend repariert.

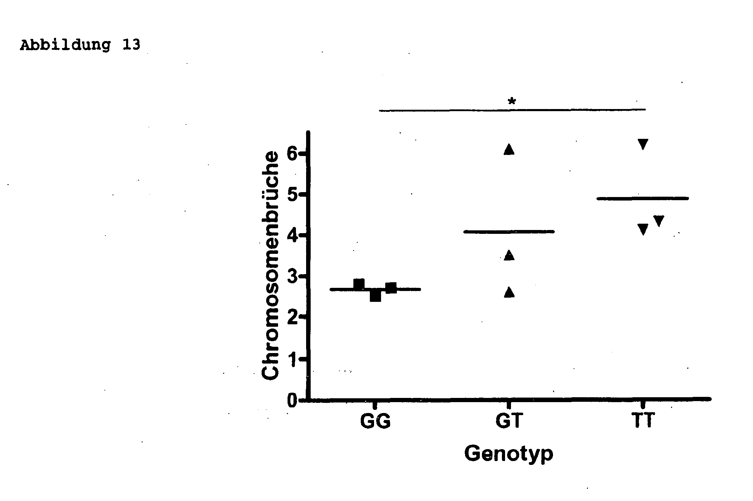

Um experimentell zu belegen, da.ss es einen Zusammenhang zwischen *CHK1*-Polymorphismen und der G2-Checkpoint-Aktivität und damit auch mit DNA-Reparaturmechanismen gibt, wurden Lymphozyten von gesunden Probanden kultiviert und zur Zellteilung angeregt. Nach 72 Stunden wurden diese Zellen mit einer Dosis von 1 Gy bestrahlt und anschließend durch den Mitosehemmer Colchicin in der M-Phase arretiert. Aus diesen Zellen wurden mittels dem Fachmann geläufiger Methoden Chromosomen präpariert und auf Schädigungen durch die Bestrahlung in jeweils 50 Metaphasen abhängig vom -1143G>T-Polymorphismus evaluiert. Auf diese Weise wurden nur die Zellen in die Auswertung einbezogen, die sich zum Zeitpunkt der Bestrahlung in der G2-Phase befanden und bis zur Chromosomenpräparation in die M-Phase gelangt sind. Somit kann durch diese Technik die Aktivität des G2/M-Checkpoints bewertet werden. Wie Abbildung 13 darstellt, beträgt die durchschnittliche Anzahl der Chromosomenbrüche pro Metaphase beim GG-Genotyp 2,7, beim GT-Genotyp 4,1 und beim TT-Genotyp 4,9 (p=0,031). Somit konnte gezeigt werden, dass mit dem GG-Genotyp, der die meiste mRNA bildet, der G2/M-Checkpoint am aktivsten ist und die entsprechenden DNA-Reparaturmechanismen am besten funktionieren konnten. Dagegen erlaubt der schwache Checkpoint beim TT-Genotyp nur wenigen Reparaturmechanismen zu arbeiten und mehr Schäden können sich ansammeln.

Damit erlaubt eine Bestimmung des Vorliegens von Polymorphismen in *CHK1* die Diagnostik der Wirksamkeit und unerwünschten Wirkungen von Arzneimitteln, insbesondere Zytostatika, sowie anderer Therapieformen, die das Erbgut der Tumorzellen schädigen, z.B. Bestrahlung. Daneben können solche Polymorphismen in *CHK1* dazu verwendet werden, die Wirkungen von Pharmaka zu diagnostizieren, die mit einem CHK1-Inhibitor kombiniert werden. Zusätzlich kann die Diagnostik des Allel- oder Haplotypstatus in *CHK1* dazu eingesetzt werden, die individuell optimale und verträgliche Dosierung von Arzneimitteln zu ermitteln.
Zur Diagnostik einer gesteigerten oder reduzierten Aktivierbarkeit der Checkpointkinase 1 und dem G2-Checkpoint dient insbesondere der Nachweis der hier beschriebenen CHK1-Promotorpolymorphismen, entweder alleine oder in allen denkbaren Kombination.

Daneben können zur Diagnostik alle weiteren Genveränderungen in *CHK1* verwendet werden, die in einem Kopplungsungleichgewicht zu diesen Polymorphismen stehen und/oder den alternativen Spleißvorgang oder die Expression zusätzlich fördern oder hemmen.

Diese Genveränderungen können mit den zuvor beschriebenen, dem Fachmann geläufigen Verfahren nachgewiesen werden. Ferner können diese Genpolymorphismen gleichzeitig nach Mulitplex-PCR und Hybridisierung an einen DNA-Chip detektiert werden. Daneben können zur Diagnostik auch andere Verfahren eingesetzt werden, die den direkten Nachweis des Expressionsniveaus von CHK1 oder Spleißvarianten von CHK1 ermöglichen.

Das genannte Verfahren eignet sich insbesondere zur Diagnostik der Wirkung von Substanzen, die die DNA der Tumorzellen schädigen. Zu diesen Substanzen gehören Oxaliplatin, 5-Fluoruracil, Folinsäure, Irinotecan, Capecitabin und Cisplatin, wobei die Liste beliebig verlängert werden kann. Vorhergesagt werden können daneben die Wirkungen von Immuntherapeutika (z.B. Interferone oder Interleukine) bzw. Inhibitoren der Signaltransduktion in Tumorzellen.

Vorhergesagt werden können ferner die Wirkungen von radiotherapeutischen Maßnahmen, wie Gamma-, Röntgenstrahlung, Elektronen, Neutronen, Protonen und Kohlenstoffionen, wobei die Liste beliebig fortgeführt werden kann. Im weiteren Sinne wird unter Strahlentherapie auch die medizinische Anwendung von Mikrowellen- und Wärmestrahlen, die Licht- und UV-Therapie sowie die Behandlung mit Ultraschallstrahlung verstanden.

Ein Beleg für die allgemeine Verwendbarkeit der *CHK1-*Polymorphismen zur Vorhersage von Arzneimittelwirkungen ergibt sich aus den beobachteten Genotypen und deren abhängigen Krankheitsverläufen bei den oben genannten Beispielen. Patienten, deren Tumore intensiv chemo- bzw. radiotherapeutisch behandelt wurden, zeigen einen günstigeren Krankheitsverlauf, wenn sie Genotyp-abhängig weniger *CHK1*-Expression aufweisen. Durch die geringere Menge an CHK1-Protein ist der G2-Checkpoint weniger aktivierbar und Zytostatika bzw. Bestrahlung können effektiver wirken. Dagegen verläuft die Erkrankung günstiger bei Tumor-Patienten, die andere therapeutische Maßnahmen erhalten haben, wenn sie vom Genotyp abhängig mehr *CHK1*-Expression zeigen. Durch die höhere Menge an CHK1-Protein ist der G2-Checkpoint stärker aktiv und kann somit zu DNA-Reparaturmechanismen beitragen und die genomische Instabilität des Tumors begrenzen.

Ein wesentlicher Gegenstand der Erfindung ist die Bereitstellung diagnostisch relevanter Genveränderungen im Gen CHK1 als Prognosefaktor für alle Tumorerkrankungen des Menschen. Naturgemäß können hierbei nicht alle Tumorerkrankungen beschrieben werden. Das Prinzip wird daher an ausgewählten Beispielen weiter erläutert, welche die generelle Verwendbarkeit demonstrieren, ohne den Schutzumfang auf die beispielhaften Ausführungsformen zu beschränken.

### Beispiele

### Beispiel 1 - Harnblasenkarzinom

Blasenkrebs ist ein bösartiger Tumor der Schleimhaut der Harnblase und tritt am häufigsten zwischen dem 60. und 70. Lebensjahr auf. Männer sind davon dreimal so häufig betroffen wie Frauen. Bei Männern ist Blasenkrebs nach Lungen- und Prostatakrebs die dritthäufigste Krebsform. Blasenkrebs kann durch äußere Einflüsse hervorgerufen werden. Zu den Risikofaktoren gehören, Rauchen, ständige Belastung des Organismus durch Chemikalien, wie beispielsweise Farbstoffe oder Schmerzmittelmissbrauch. Bei vielen Patienten ergeben die Untersuchungen, dass es sich um einen oberflächlichen Tumor handelt. Dieser kann operativ mit Hilfe des Zystoskops entfernt werden. Mehr als 70% der Patienten, die wegen eines oberflächlichen Blasenkarzinoms behandelt wurden, zeigen im Verlauf ein Tumorrezidiv. Dabei kommen in mehr als der Hälfte Rezidivtumoren mit nicht-muskelinvasiver Erkrankung vor. Diese können durch transurethrale Resektion kurativ behandelt bzw. kontrolliert werden. Es ist deshalb wichtig, diese Läsionen frühzeitig zu erkennen und den Patienten regelmäßig und engmaschig nachzusorgen. In regelmäßigen Intervallen dienen Ausscheidungsurogramme zur Kontrolie möglicher Tumormanifestationen in Nierenbecken und Harnleitern. Bislang gibt es kaum valide Marker, die für den weiteren Verlauf der Erkrankung prädiktiv sind. Derzeit werden daher die klassischen Faktoren wie Eindringtiefe, Differenzierungsgrad, Metastasierung, Lymphknotenbefall etc. für die Prognose herangezogen. Genetische Marker für Überlebenswahrscheinlichkeit und Therapieansprechen würden die Betreuung von Patienten mit Harnblasenkarzinom wesentlich verbessern. Aufgabe der Erfindung ist es zu zeigen, dass die Verwendung von Genveränderungen in *CHK1* dazu geeignet ist, den weiteren Verlauf der Erkrankung vorherzusagen.

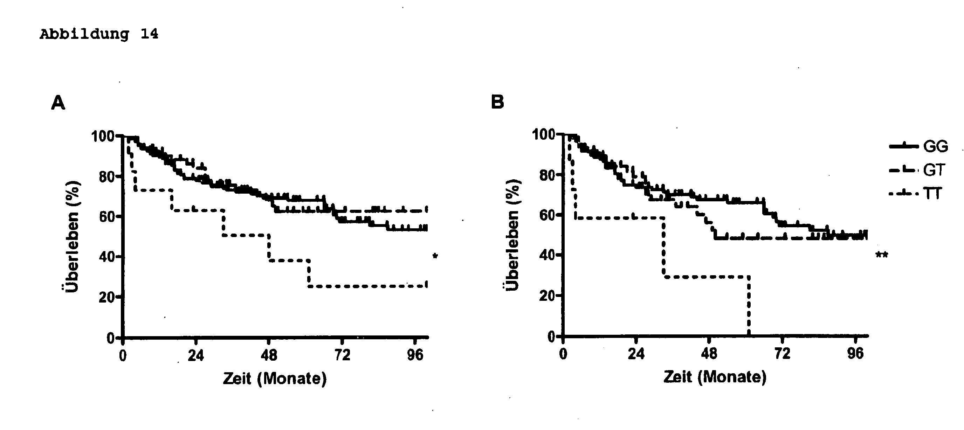

Abbildung 14A zeigt das Überleben in Abhängigkeit vom - 1143G>T-SNP. Hierbei ist das Risiko für das Versterben bei Patienten mit dem TT-Genotyp um ca. das 2-fache erhöht (p=0.042). Die mediane Zeit bis zum Tod beträgt nur 48 Monate bei Trägern des TT-Genotyps während bei G-Allel-Trägern keine mediane Zeit angegeben werden kann, da bis zum Ende der Studie weniger als die Hälfte dieser Patienten verstarben. Eine ähnliche Beziehung wird gefunden, wenn nur das Überleben der älteren Patienten untersucht wird (Abbildung 14B). Der Kurvenverlauf ist für die Genotypen signifikant verschieden (p=0.004), wobei den G-Allelträgern der günstigere Verlauf zugeordnet wird. Die Überlebenszeit beträgt im Median 87 Monate (GG-Genotyp) bzw. 50 Monate (GT-Genotyp) bei Patienten mit dem G-Allel, bei den homozygoten T-Allelträgern dagegen nur 33 Monate.

### Beispiel 2 - Kolorektales Karzinom

Das kolorektale Karzinom ist die häufigste Tumorart im Gastrointestinalträkt und eine der Hauptursachen für einen Tumor-bedingten Tod weltweit (12-15% der gesamten Krebsmortalität). In Deutschland beträgt die Inzidenz etwa 51.000 Neuerkrankungen pro Jahr. Die mittlere 5-Jahres-Überlebensrate nach der Tumorresektion beläuft sich auf nur ca. 50%. Ernährungsgewohnheiten, Krebsfördernde Metabolite, exogene Karzinogene und bestimmte prädisponierende Erkrankungen zählen zu den Risikofaktoren für das Entstehen eines kolorektalen Karzinoms. Die Standardmethode zur Vorhersage des Krankheitsverlaufes ist das TNM- bzw. UICC-Stadien-System. Patienten mit den UICC-Stadien III oder IV haben generell eine schlechtere Prognose als Patienten mit den UICC-Stadien I oder II. Eine adjuvante Chemotherapie wird bei metastasierten kolorektalen Karzinomen (UICC-Stadien III und IV) durchgeführt und kann den lokalen Effekt einer Strahlentherapie verstärken. Ein Großteil dieser Patienten entwickelt Rezidive oder Metastasen, was eine intensive Nachsorge erforderlich macht. Somit ist es wichtig, molekulare Marker zu identifizieren und zu etablieren, die den weiteren Verlauf der Erkrankung vorhersagen können. Ein weiterer Bestandteil der Erfindung besteht darin, Genveränderungen in *CFIK1* zu nutzen, um den weiteren Verlauf des kolorektalen Karzinoms vorherzusagen.

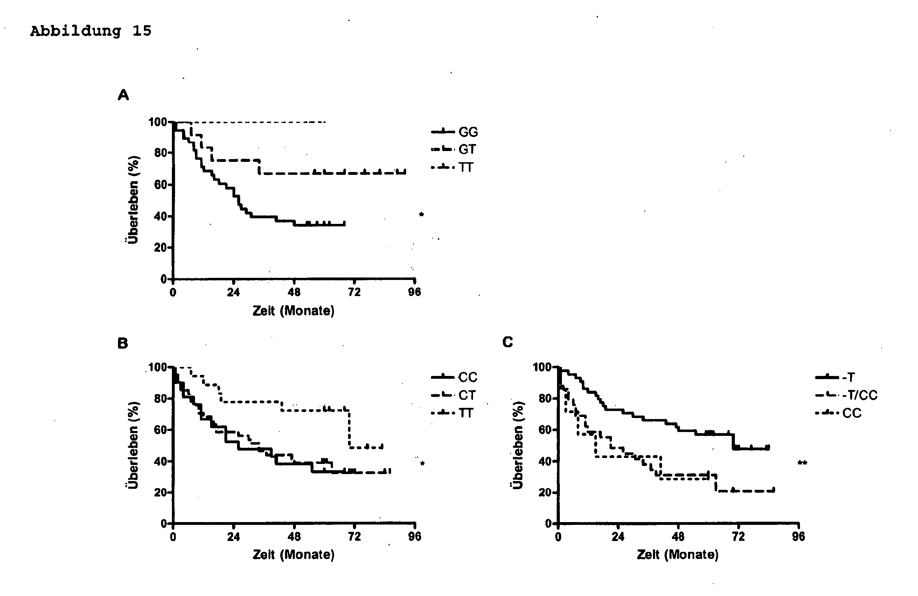

Abbildung 15A zeigt einen signifikanten Unterschied bezüglich des Überlebens in Abhängigkeit vom -1143G>T-Polymorphismus (p=0,026). Patienten mit dem GG-Genotyp überleben im Median 26 Monate, wohingegen bei den T-Allelträgern während des Beobachtungszeitraumes weniger als die Hälfte der Patienten stirbt. Zudem ist ein Gendosiseffekt erkennbar, da Träger des heterozygoten Genotyps ein höheres Risiko als der TT-Genotyp und ein geringeres Risiko als der GG-Genotyp aufweisen. In Abbildung 15B ist in Abhängigkeit des -1400C>T-SNPs das Überleben der Patienten mit Tumorlokalisation im Kolon dargestellt. Träger des TT-Genotyps überleben signifikant länger als Träger des C-Allels (p=0.033). Während das mediane Überleben beim TT-Genotyp 70 Monate beträgt, sind dies nur 34 Monate beim CT-Genotyp und nur 26 Monate beim CC-Genotyp. Abhängig von den Genotypen der Polymorphismen -1453T>C und -1454insC ist ebenfalls ein signifikanter Unterschied bezüglich Überleben zu erkennen (Abbildung 15C). Patienten die an Position -1453 den TT-Genotyp und an Position -1454 keine Insertion haben, überleben länger als Patienten, die das -1454C-Allel und die Insertion besitzen (p=0.007). Das mediane Überleben beträgt 70 Monate für den TT-Genotyp ohne Insertion im Vergleich zu 21 und 15 Monaten für die anderen Genotypen.

Interessant ist hier vor allem die Beobachtung, dass Patienten, die eine geringe CHK1-mRNA-Expression besitzen, da sie Träger des TT-Genotyps sind, am längsten überleben. Da Patienten mit den UICC-Stadien III und IV, die den größten Teil dieses Kollektivs ausmachen, eine intensive Behandlung gegen ihre Tumore erhalten haben, fällt dem Umstand länger zu überleben, wenn man wenig CHK1-Protein besitzt, eine große Bedeutung zu. Reduziertes CHK1 wird zwar als Prädisposition für Tumorerkrankungen angesehen, macht die Tumorzellen aber auch empfänglicher gegenüber therapeutischen Maßnahmen. Dies können die hier gezeigten Überlebenskurven bestätigen.

### Beispiel 3 - Chronische lymphatische Leukämie

Bei der chronischen lymphatischen Leukämie (CLL) handelt es sich um eine chronisch verlaufende Form einer Leukämie. Charakteristisch für die Krankheit ist eine große Zahl von entarteten Lymphozyten. Insgesamt 30 Prozent aller leukämischen Erkrankungen sind chronisch lymphatische Leukämien. Das mittlere Erkrankungsalter liegt bei 65 Jahren. Eine CLL kann bis zu 20 Jahre lang gutartig verlaufen, das heißt, die Patienten zeigen außer vergrößerten Lymphknoten, Müdigkeit und Appetitlosigkeit keine Symptome. Die Behandlung setzt erst dann ein, wenn die Zahl der Lymphozyten stark ansteigt, der Anteil der Erythrozyten und Thrombozyten absinkt oder andere Komplikationen auftreten. Eine frühzeitige Behandlung hat keinen Einfluss auf den Verlauf der Erkrankung. Die wichtigste therapeutische Maßnahme ist die Chemotherapie. Je weiter die Erkrankung fortgeschritten ist, desto größer sind die Gesundheitsstörungen durch die Veränderung des Organsystems. Je nach Binet-Stadium der Krankheit kann der Arzt die Prognose abschätzen. Das Stadium einer CLL ist unter anderem dadurch gekennzeichnet, wie viele Lymphozyten sich im Blut und Knochenmark befinden, wie groß Milz und Leber sind und ob eine Anämie vorliegt. Eine CLL führt zu Veränderungen im Immunsystem, so dass Menschen, die an dieser Krankheit leiden, stärker gefährdet sind, andere Arten von Krebs zu entwickeln. Bei gleichem Binet-Stadium zeigen Patienten jedoch ganz unterschiedliche Krankheitsverläufe. Aufgabe der Erfindung ist es zu zeigen, dass Genveränderungen im Gen *CHK1* geeignet sind, den Verlauf der CLL vorherzusagen.

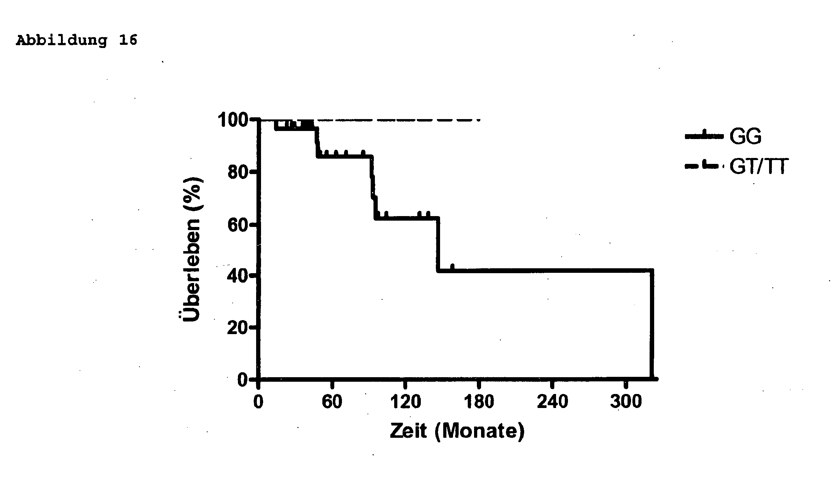

Hierzu wurden Patienten mit CLL bezüglich der beschrieben Genveränderungen in *CHK1* genotypisiert und der Genstatus mit dem überleben assoziiert. Abbildung 16 zeigt das Überleben abhängig vom -1143G>T-Genotyp. Patienten, die Träger des T-Allels sind, überleben länger als Patienten, die homozygot GG sind. Beim GG-Genotyp beträgt das mediane Überleben 146 Monate, dagegen stirbt aber weniger als die Hälfte der Patienten während des Beobachtungszeitraumes, die das T-Allel tragen. Auch hier zeigt sich, dass unter intensiver Behandlung ein Genotyp, der zu einer geringen *CHK1*-Expression führt, für das Überleben am günstigsten ist.

### Beispiel 4 - Malignes Melanom

Das maligne Melanom ist eine bösartige Entartung der Melanozyten (Pigmentzellen der Haut), weshalb es auch den Namen "schwarzer Hautkrebs" trägt. Diese Tumorart neigt dazu, sehr früh Metastasen über die Blut- und Lymphbahnen zu streuen. Die Inzidenz des malignen Melanoms ist steigend, sie verdoppelt sich alle 15 Jahre. Gefährdet sind besonders Personen mit geringer Pigmentierung. Zu den Risikofaktoren zählen v. a. intensive Sonnenexposition und eine Sonnenbrandanamnese von 5 oder mehr Episoden in der Jugend. In der westlichen Welt ist das maligne Melanom der häufigste Krebs von Frauen zwischen 20 und 40 Jahren. Kriterien zur Prognose und Therapie liefern die Stadien der TNM-Klassifikation, die Tumordicke nach Breslow, die Eindringtiefe nach Clark, die Unterscheidung nach Subtypen und Lokalisation. Bei einer frühzeitigen Diagnose und Behandlung stehen die Heilungschancen noch gut. Bei spät diagnostizierten Melanomen mit Lymphknotenmetastasen liegt die 5-Jahres-Überlebensrate bei ca. 30%, sind bereits Fernmetastasen vorhanden, beträgt sie nur 0-5%. Molekulare Marker für den Verlauf dieser Erkrankung und Therapieansprechen gibt es nicht. Die Identifizierung solcher Marker würde die Vor- und Nachsorge der Patienten stark verbessern.

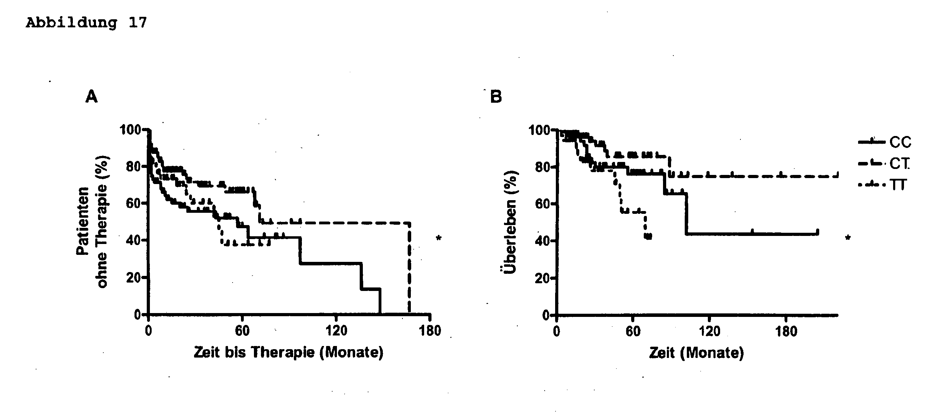

Abbildung 17A zeigt den Genotyp-abhängigen Unterschied des -1400C>T-SNP auf die Zeit von Erstdiagnose und Ersttherapie bis zum Zeitpunkt der Erfordernis einer weiteren Therapie. Patienten, die Träger mindestens eines C-Allels sind, beginnen die weiterführende Therapie signifikant später als Patienten mit dem TT-Genotyp (p=0.033). Für den heterozygoten bzw. homozygoten C-Allel-Träger wird die Therapie im Median nach 71 bzw. 57 Monaten notwendig, beim TT-Genotyp schon nach 45 Monaten. Hier zeigt sich im Gegensatz zu den bisher diskutierten Kurvenverläufen, dass ohne medizinische Maßnahmen, die Genotypen begünstigt sind, die eine hohe *CHK1*-Expression und damit sehr gut funktionierende Checkpoints aufweisen. Abbildung 17B gibt das Überleben aller Patienten wider. Auch hier ist zu erkennen, dass Patienten, die mindestens ein C-Allel tragen, länger überleben als Patienten mit dem TT-Genotyp (p=0.013). Das mediane Überleben beträgt beim TT-Genotyp nur 69 Monate, dagegen beim CC-Genotyp 101 Monate und der heterozygote Genotyp erreicht die mediane Überlebenszeit während des Beobachtungszeitraumes gar nicht. Bei dieser Tumorart erfolgt die Therapie des Primärtumors rein chirurgisch. Nach der Exzision des Tumors werden weitere Therapien in Erwägung gezogen. Bei 76,5% der Patienten erfolgte keine weitere Therapie, 11,2% erhielten eine Immuntherapie mit Interferon-α, 3,4% eine hypertherme Extrimitätenperfusion, bei 5,4% war eine Nachresektion erforderlich und die restlichen 3,5% unterzogen sich anderen therapeutischen Maßnahmen. Da nur 1,6% der Patienten eine Chemo- und/oder Radiotherapie erhielten, die von einer geringen *CHK1*-Expression begünstigt wäre, sind hier die Genotypen bevorteiligt, die eine starke *CHK1-*Expression (Real time-PCR Ergebnisse aus Abb. 12B: CC-und CT-Genotyp-0.009±0.002, TT-Genotyp 0.004±0.002, p=0.049) besitzen mit einer stark funktionierenden Checkpointkinase 1.

### Beispiel 5 - Cholangiozelluläres Karzinom

Das cholangiozelluläre Karzinom (CCC) ist ein maligner Tumor der Gallengänge der Leber. Im Vergleich zu Asien und Afrika, wo es die häufigste Tumorart mit 20-30% der malignen Tumore darstellt, ist es in Mitteleuropa relativ selten (<1% aller bösartigen Erkrankungen). Zu den Risikofaktoren zählen Colitis Ulcerosa, chronische Gallengangsentzündungen und virale Hepatitiden. Die kurative Behandlung eines CCC erfolgt durch eine Leberteilresektion oder eine totale Hepatektomie mit Lebertransplantation. Die Rezidivrate ist sehr hoch. Die Prognose eines CCC ist daher sehr ungünstig, besonders bei nicht-resektablen Tumoren, die eine 5-Jahres-Überlebensrate von <10% haben. Die mittlere Überlebensdauer bei Patienten mit nichtresektablem CCC beträgt 6 bis 10 Monate. Molekulare Marker sind bisher für den Verlauf dieser Erkrankung noch nicht bekannt, würden aber die Betreuung von Patienten mit CCC wesentlich verbessern. Bestandteil der Erfindung ist es deshalb zu zeigen, dass die Verwendung von Genveränderungen in *CHK1* dazu geeignet ist, den weiteren Verlauf der Erkrankung vorherzusagen.

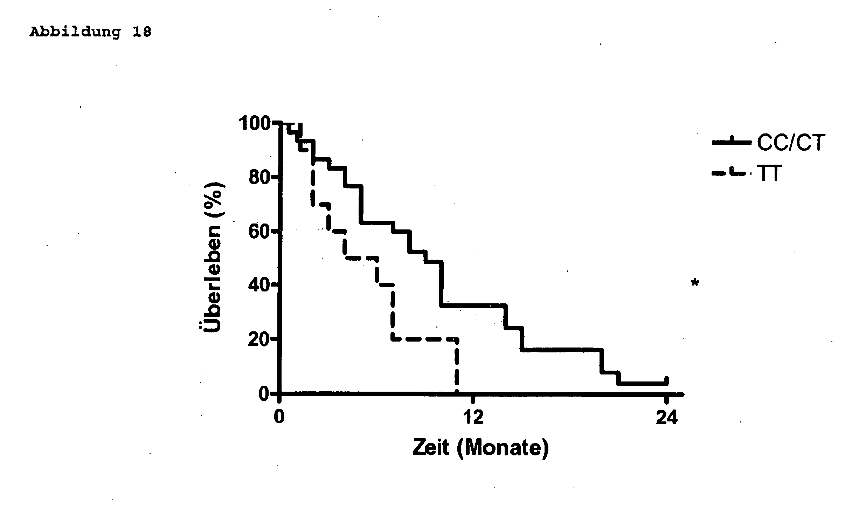

Abbildung 18 zeigt das Überleben von CCC-Patienten. Patienten mit dem CC- und CT-Genotyp überleben signifikant länger als Patienten mit dem TT-Genotyp (p=0.036). Das mediane Überleben des TT-Genotyps beträgt 5 Monate, das der anderen Genotypen 9 Monate. Da die Überlebenswahrscheinlichkeit der CCC-Patienten nur sehr gering ist, ist die Zeit für eine intensive Therapie nach der Operation sehr kurz. Wegen der meist vorgeschädigten zirrhotischen Leber ist die Indikation zur Radiotherapie nur selten gegeben, zudem ist die Leber äußerst radiosensitiv. So ist bis zum Erreichen einer tumoriziden Dosis die Leber evtl. bereits zerstört. Die systemische Chemotherapie mit zahlreichen Zytostatika und Kombinationen hat bisher keine nachdrückliche Effektivität gezeigt. Die Remissionsraten liegen bei 20 bis 30%, die mittlere Remissionsdauer beträgt 4 bis 6 Monate. Alternativ können Chemoembolisationen oder gezielte intratumorale Alkoholinjektionen angewendet werden. Da beim CCC weder Radio- noch Chemotherapie in ausreichendem Maße eingesetzt werden können, die bei einem niedrigem CHK1-Level Vorteile aufzeigen würden, sind es bei dieser Tumorart die Genotypen, die eine vermehrte *CHK1*-Expression zeigen, die ein längeres Überleben aufweisen.

### Beispiel 6 - Mammakarzinom

Das Mammakarzinom ist der häufigste Tumor der weiblichen Bevölkerung in Europa und den USA. Es betrifft 7-10% aller Frauen und macht 25% der gesamten weiblichen Krebsmortalität aus. Die Ätiologie des Mammakarzinoms ist noch nicht bekannt, jedoch sind Risikofaktoren beschrieben, wie eine familiäre Disposition, Strahlenexposition oder Östrogeneinfluss. Bei den meisten Patientinnen ergeben die Untersuchungen, dass es sich um ein invasives Karzinom handelt. Mit wenigen Ausnahmen wird jedes operable Mammakarzinom selbst nachgewiesener Fernmetastasierung chirurgisch behandelt. Die unterschiedlich radikale operative Erstversorgung hat Variationen der lokoregionären Rezidivrate, nicht aber der langfristigen Überlebenschance zur Folge. Zudem können Rezidive oder Fernmetastasen nicht selten erst nach 5 oder sogar 10 Jahren manifest werden. Es ist deshalb wichtig, diese Läsionen frühzeitig zu erkennen und die Patientinnen engmaschig nachzusorgen. Nachsorgeuntersuchungen werden in regelmäßigen Intervallen durchgeführt, bei zwischenzeitlichem Verdacht auch bis zu 10 Jahre postoperativ. Bislang gibt es kaum valide Marker, die für den weiteren Verlauf der Erkrankung prädiktiv sind. Derzeit werden daher die klassischen Faktoren wie Tumorgröße, Metastasierung, Lymphknotenbefall, Hormonrezeptorstatus etc. für die Prognose herangezogen. Genetische Marker für Überlebenswahrscheinlichkeit und Therapieansprechen würden die Betreuung von Patientinnen mit Mammakarzinom wesentlich verbessern. Aufgabe der Erfindung ist es zu zeigen, dass die Verwendung von Genveränderungen in *CHK1* dazu geeignet ist, den weiteren Verlauf der Erkrankung vorherzusagen.

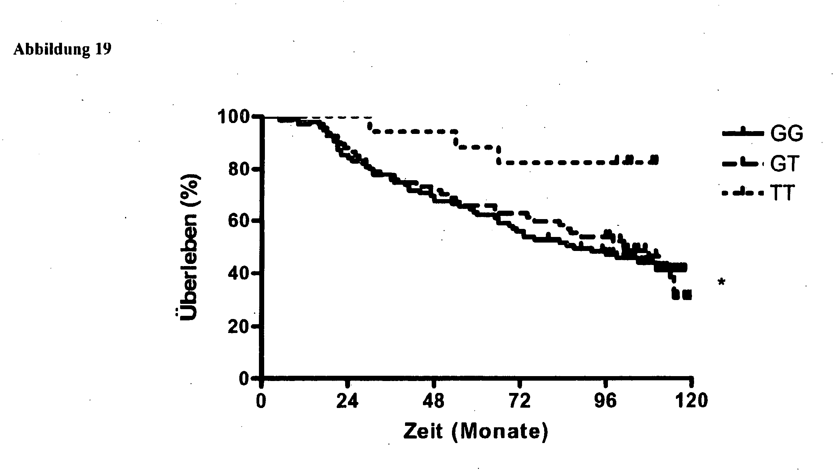

Abbildung 19 zeigt einen signifikanten Unterschied bezüglich des Überlebens in Abhängigkeit vom -1143G>T-Polymorphismus (p=0,017). Patienten mit dem GG-Genotyp überleben im Median 87 Monate, wohingegen bei den Patienten mit dem GT-Genotyp die mediane Überlebenszeit 101 Monate beträgt. Im Gegensatz dazu stirbt bei homozygoten T-Allelträgern während des Beobachtungszeitraumes weniger als die Hälfte der Patienten.

Interessant ist hier vor allem wieder die Beobachtung, dass Patienten, die eine geringe CHK1-mRNA-Expression besitzen, da sie Träger des TT-Genotyps sind, am längsten überleben. Patientinnen mit Mammakarzinom erhalten neben einer möglichen Hormontherapie in den meisten Fällen eine adjuvante Radio- und/oder Chemotherapie. Dies bedeutet, dass eine reduzierte CHK1-Proteinmenge in Zusammenhang mit einer solchen Therapiestrategie zu einem längeren Überleben bei den Patientinnen führt.

### Andere Erkrankungen

Während sich die hier dargestellten Beispiele ausnahmslos auf Krebserkrankungen beziehen, so ist doch zu betonen, dass Proliferationsvorgänge, Apoptose und zellulärer Umbau bei allen Erkrankungen des Menschen vorkommen und dass damit bei diesen Vorgängen DNA-Reparaturmechanismen und DNA-Checkpoints eine große Rolle spielen. Beispielsweise entwickelt sich der Herzinfarkt auf dem Boden von Gefäßveränderungen der Koronararterien, bei denen proliferative Vorgänge im Vordergrund stehen. Auch die Erholung des Myokards nach Infarkt erfordert solche Umbauvorgänge. Gleiches gilt für das Gehirn nach einem ischämischen Infarkt. Insofern können damit eine oder mehrere der hier beschriebenen Genveränderungen dazu verwendet werden, den Verlauf von Herz-Kreislauf Erkrankungen vorherzusagen. Auch bein Infektionserkrnakungen wie z.B. mit dem Hepatitisvirus erfolgen solche Proliferationsvorgänge, die beispielsweise bei der Leber zur Zirrhose führen können. Insofern können damit eine oder mehrere der hier beschriebenen Genveränderungen dazu verwendet werden, den Verlauf von Infektionserkrankungen vorherzusagen. Auch bei neurodegenerativen Erkrankungen, z.B. Morbus Alzheimer oder multiples Sklerose, spielen Wachstums- und zelluläre Umbauprozess eine große Rolle.

## Patentansprüche

1. In vitro-Verfahren zur Vorhersage des Krankheitsverlaufs einer Krebserkrankung, **dadurch gekennzeichnet, dass** man nach einer oder mehreren Genveränderung/en in der Promotorregion des Gens CHK1 (CHEK1) auf dem Humanchromosom 11q23 (Accession NO. NM_033899 NCBI) sucht und die Genveränderung ausgewählt ist aus (-1143)G>T, (-1400)C>T, (-1453)T>C und (-1454)InsC.

2. In vitro-Verfahren zur Vorhersage des Ansprechens einer Radiochemotherapie bei einer Krebserkrankung **dadurch gekennzeichnet, dass** man nach der Genveränderung (-1143)G>T in der Promotorregion des Gens CHK1 (CHEK1) auf dem Humanchromosom 11q23 (Accession No. NM_033899 NCBI) sucht.

## Claims

1. An in vitro method for the prediction of the course of a cancer disease, **characterised in that** one or more gene modification/s are searched for in the promoter region of the gene CHK1 (CHEK1) on the human chromosome 11 q23 (accession no. NM_033899 NCBI) and the gene modification is selected from (-1143) G>T, (-1400) C>T, (-1453) T>C and (-1454) InsC.

2. An in vitro method for the prediction of the response to a radiochemotherapy of a cancer disease **characterised in that** a gene modification (-1143) G>T is searched for in the promoter region of the gene CHK1 (CHEK1) on the human chromosome 11q23 (accession no. NM_033899 NCBI).

## Revendications

1. Procédé in vitro pour prédire l'évolution de maladie d'une maladie cancéreuse, **caractérisé en ce qu'**on recherche une ou plusieurs modifications géniques dans la région du promoteur du gène CHK1 (CHEK1) sur le chromosome humain11q23 (n°. d'ordre NM_033899 NCBI) et la modification génique est choisie parmi (-1143) G > T, (-1400) C > T, (-1453) T > C et (-1454) InsC.

2. Procédé in vitro pour prédire la réponse à une radiochimiothérapie dans une maladie cancéreuse **caractérisé en ce qu'**on recherche la modification génique (-1143) G > T dans la région du promoteur du gène CHK1 (CHEK1) sur le chromosome humain 11q23 (n°. d'ordre NM_033899 NCBI).
